# EUROPEAN PATENT APPLICATION

(11) **EP 4 299 587 A1**
(43) Date of publication of application: **03.01.2024**
(21) Application number: 22775609.5
(22) Date of filing: 22.03.2022
(51) Int. Cl.: C07K 16/18, G01N 33/53, G01N 33/68, C07K 14/775

(54) **METHOD AND KIT FOR ASSISTING IN DETERMINATION OF MALIGNANT PANCREATIC CYSTIC TUMOR**

(30) Priority: 24.03.2021 JP 2021049931
(71) Applicant: Toray Industries, Inc., Tokyo 103-8666 (JP); National Cancer Center, Tokyo 104-0045 (JP)
(72) Inventor: KOBAYASHI Michimoto, Tokyo 103-8666 (JP); KAWAI Hideki, Tokyo 103-8666 (JP); SOGAME Asako, Tokyo 103-8666 (JP); HONDA Kazufumi, Tokyo 104-0045 (JP)
(74) Representative: Prüfer & Partner mbB Patentanwälte · Rechtsanwälte
(86) International application number: PCT/JP2022/013234
(87) International publication number: WO 2022/202826

(57) **Abstract**

The present invention provides a convenient and noninvasive detection method for assisting in the determination of pancreatic cystic tumor to be benign or malignant, and a kit for detection therefor. Specifically, the present invention provides a method for assisting in the determination of malignant pancreatic cystic tumor, the method comprising the steps of measuring *in vitro* the amount of APOA2-AT protein or/and APOA2-ATQ protein present in a body fluid sample of a test subject having pancreatic cystic tumor, and assisting in determining the pancreatic cystic tumor to be benign or malignant on the basis of the amount, and a kit for assisting in the determination of malignant pancreatic cystic tumor, the kit being directed to measuring the amount of APOA2-AT protein or/and APOA2-ATQ protein and being usable in the method.

## Description

### Technical Field

The present invention relates to a method for detecting malignant pancreatic cystic tumor, comprising measuring the amounts of APOA2 protein variants in a body fluid sample of a test subject having pancreatic cystic tumor, and a kit for the detection of malignant pancreatic cystic tumor, comprising anti-APOA2 antibodies.

### Background Art

Pancreatic cystic tumor is a lesion having a saclike structure (cyst) that retains a liquid inside a tumor tissue. In recent years, the frequency with which pancreatic cystic tumor is accidentally found in daily medical practice has increased with advancement in imaging techniques. Although pancreatic cystic tumor progresses slowly and is asymptomatic in many cases, there is a possibility of becoming malignant with time, developing pancreatic cancer. Therefore, pancreatic cystic tumor needs to be regularly tested for whether to benign or malignant by follow-up.

Various imaging techniques such as CT (computerized tomography), MRI (magnetic resonance imaging), MRCP (magnetic resonance cholangiopancreatography), and EUS (endoscopic ultrasound) are used in the detection of pancreatic cystic tumor. However, pancreatic cystic tumor is difficult to determine to be benign or malignant even by the latest testing techniques (Non Patent Literatures 1 and 2). Although EUS is reportedly effective for the detection of a malignant lesion, problems thereof are the dependence of results on the skills of a laboratory technician and relatively large invasiveness. An attempt is also made on a testing method for detecting a tumor marker CEA in cystic fluid by EUS-FNA (endoscopic ultrasound-fine needle aspiration). Reportedly, a high CEA level is effective for the determination of a mucinous lesion and a nonmucinous lesion and however, cannot be used in the determination of a malignant lesion and a benign lesion. Likewise, cytology of detecting cancer cells in cystic fluid using EUS-FNA is regarded as a testing method that can obtain definitive diagnosis as a malignant lesion and however, is not recommended in Japan for reasons such as the risk of peritoneal dissemination caused by the leakage of cystic fluid. In addition, there is cytology using pancreatic juice collected during endoscopic retrograde cholangiopancreatography (ERCP). This method, albeit effective for the detection of a malignant lesion, involves the risk of onset of pancreatitis, etc. at the time of diagnosis (Non Patent Literatures 1 and 2). The development of a novel testing technique that can determine pancreatic cystic tumor to be benign or malignant with high accuracy by a noninvasive method is currently demanded for medical sites.

The APOA2 (apolipoprotein A2 or apolipoprotein A-II) protein (GenBank Accession No. NP_001634.1) is a member of the apolipoprotein family constituting serum lipoproteins. Ten or more apolipoproteins have heretofore been known, and their main functions are, for example, the structural stabilization of the lipoproteins, the activation of enzymes involved in lipoprotein metabolism, and effects as ligands for lipoprotein receptors present on cell surface. The APOA2 protein is synthesized as a 100-amino acid precursor comprising a signal peptide in a liver tissue. Its mature form present in blood consists of 77 amino acids. The mature form of the APOA2 protein is a high-density lipoprotein (HDL)-constituting apolipoprotein having a glutamine residue (Q) at its amino terminus (N terminus), a threonine residue (T) at the 76th position counted from the N terminus, and glutamine residue (Q) at the carboxyl terminus (C terminus) (77th position counted from the N terminus). Also, the APOA2 protein has been reported to have variants differing in mass, including APOA2-ATQ protein (full-length APOA2 protein), APOA2-AT protein (APOA2 protein lacking the C-terminal glutamine residue (Q)), and APOA2-A protein (APOA2 protein lacking the C-terminal glutamine and threonine residues (QT)) (Non Patent Literature 3).

According to analysis based on the conformational data of the APOA2 protein (PDB ID: 1L6L) registered in the protein structure data bank (PDB; Protein Data Bank; http://www.rcsb.org/pdb/home.do), the APOA2 proteins are dimerized through the disulfide bond (SS-bond) between their cysteine residues on the N-terminal side. Thus, the APOA2 protein has been found to exist in blood as dimers having various molecular weights resulting from the combinations of the 3 variants. These dimers are known to include, for example, a dimer consisting of the full-length APOA2-ATQ proteins (APOA2-ATQ/ATQ protein dimer), a dimer of the APOA2-ATQ protein and the APOA2-AT protein (APOA2-ATQ/AT protein dimer), a dimer consisting of the APOA2-AT proteins (APOA2-AT/AT protein dimer), a dimer of the APOA2-AT protein and the APOA2-A protein (APOA2-AT/A protein dimer), and a dimer consisting of the APOA2-A proteins (APOA2-A/A protein dimer).

These various APOA2 protein dimers are known to exhibit quantitative variations in the blood of pancreatic cancer patients compared with normal persons. Particularly, the APOA2-ATQ/AT protein dimer has been found as a protein having a mass value of molecular weight 17253 ± 9 (m/z) as a result of mass spectrometry. It has been revealed that: this protein dimer is significantly decreased in pancreatic cancer patients compared with normal persons; and pancreatic cancer can be detected with accuracy as high as an AUC (area under the curve) value of 0.85 or higher by using the protein dimer as a pancreatic cancer marker (Patent Literature 1 and Non Patent Literature 3). It is known that various pancreatic tumors including pancreatic cancer and pancreatic cystic tumor can be detected with accuracy equivalent to or higher than mass spectrometry by measuring the amounts of two types of variants constituting the various APOA2 protein dimers, and combining the measurement results (Patent Literature 2 and Non Patent Literature 4).

### Citation List

### Patent Literature

Patent Literature 1: International Publication No. WO 2006/098087
Patent Literature 2: International Publication No. WO 2015/050107

### Non Patent Literature

Non Patent Literature 1: Working Group of International Association of Pancreatology (representative: Masao Tanaka), International consensus guidelines 2017 for management of IPMN
Non Patent Literature 2: Working Group of International Association of Pancreatology (representative: Masao Tanaka), International consensus guidelines 2012 for management of IPMN/MCN
Non Patent Literature 3: Honda K. et al., 2012, PLoS One, Vol. 7, e46908
Non Patent Literature 4: Honda K. et al., 2015, Sci Rep. Vol. 5, 15921

### Summary of Invention

### Object to be Achieved

Previous testing methods for use in the determination of malignant pancreatic cystic tumor present problems such as high invasiveness and difficult learning of testing skills.

An object of the present invention is to provide a convenient and noninvasive method for assisting in the determination of malignant pancreatic cystic tumor targeting the blood of a pancreatic cystic tumor patient, and a kit for assisting in the determination of malignant pancreatic cystic tumor.

### Means to Achieve the Object

In order to attain the object, the present inventors have first studied whether two APOA2 protein variants (APOA2-AT protein and APOA2-ATQ protein) reportedly effective for assisting in the determination of pancreatic cancer or pancreatic cystic tumor are effective for discriminating malignant pancreatic cystic tumor from benign pancreatic cystic tumor. As a result, the present inventors have found that the concentration of the APOA2-AT protein in blood is lower in malignant pancreatic cystic tumor compared with benign pancreatic cystic tumor; and the concentration of the APOA2-ATQ protein in blood is higher therein. The present inventors have thereby found that pancreatic cystic tumor can be determined to be benign or malignant using the two APOA2 protein variants in a body fluid sample, leading to the completion of the present invention.

The present invention encompasses the following aspects:
(1) A method for assisting in the determination of malignant pancreatic cystic tumor in a test subject having pancreatic cystic tumor, the method comprising the steps of measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, present in a body fluid sample obtained from the test subject, and assisting in the determination of the pancreatic cystic tumor of the test subject to be benign or malignant on the basis of the amount.
(2) The method according to (1), wherein the pancreatic cystic tumor is intraductal papillary mucinous neoplasm (IPMN) or mucinous cystic neoplasm (MCN).
(3) The method according to (1) or (2), wherein the determination of the pancreatic cystic tumor to be malignant is assisted in when the amount of the APOA2-AT protein present in the body fluid sample obtained from the test subject is lower than the amount of the APOA2-AT protein present in a body fluid sample obtained from a test subject with known benign pancreatic cystic tumor.
(4) The method according to (3), wherein the determination of the pancreatic cystic tumor to be malignant is assisted in when the amount of the APOA2-AT protein present in the body fluid sample obtained from the test subject is lower than a cutoff value set on the basis of the amount of the APOA2-AT protein present in a body fluid sample obtained from a test subject with known benign pancreatic cystic tumor or a cutoff value set on the basis of the amount of the APOA2-AT protein present in a sample obtained from a test subject with known malignant pancreatic cystic tumor.
(5) The method according to any of (1) to (4), wherein the determination of the pancreatic cystic tumor to be malignant is assisted in when the amount of the APOA2-ATQ protein present in the body fluid sample of the test subject is higher than the amount of the APOA2-ATQ protein present in a body fluid sample obtained from a test subject with known benign pancreatic cystic tumor.
(6) The method according to (5), wherein the determination of the pancreatic cystic tumor to be malignant is assisted in when the amount of the APOA2-ATQ protein present in the body fluid sample obtained from the test subject is higher than a cutoff value set on the basis of the amount of the APOA2-ATQ protein present in a body fluid sample obtained from a test subject with known benign pancreatic cystic tumor or a cutoff value set on the basis of the amount of the APOA2-ATQ protein present in a sample obtained from a test subject with known malignant pancreatic cystic tumor.
(7) The method according to any of (1) to (6), wherein the body fluid sample is blood, plasma, or serum.
(8) The method according to any of (1) to (7), wherein the test subject is determined in advance to have pancreatic cystic tumor by an imaging technique and/or the following steps (A) to (C):
   (A) a first step of measuring the amount of APOA2-ATQ protein in the sample using an anti-APOA2-ATQ terminus antibody specifically binding to a carboxyl terminal region of the APOA2-ATQ protein consisting of the amino acid sequence represented by SEQ ID NO: 2, and an anti-APOA2-ATQ non-terminus antibody binding to the amino acid sequence other than the carboxyl terminal region;
   (B) a second step of measuring the amount of APOA2-AT protein in the sample using an anti-APOA2-AT terminus antibody specifically binding to a carboxyl terminal region of the APOA2-AT protein consisting of the amino acid sequence represented by SEQ ID NO: 1 and an anti-APOA2-AT non-terminus antibody binding to the amino acid sequence other than the carboxyl terminal region; and
   (C) a third step for determining the presence or absence of pancreatic cystic tumor by inputting, to a preset discriminant, the measurement value of the amount of APOA2-ATQ protein obtained in the first step and the measurement value of the amount of the APOA2-AT protein obtained in the second step, and comparing the resulting discriminant value with the discriminant value of a known normal subject.
(9) A kit for assisting in the determination of malignant pancreatic cystic tumor, the kit being directed to measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, comprised in a body fluid sample obtained from a test subject.
(10) The kit according to (9), comprising an anti-APOA2-AT terminus antibody or a binding fragment thereof, or/and an anti-APOA2-ATQ terminus antibody or a binding fragment thereof.
(11) The kit according to (10), wherein the anti-APOA2-ATQ terminus antibody or the binding fragment thereof is an anti-APOA2-ATQ terminus monoclonal antibody that specifically binds to a carboxyl-terminal region of the APOA2-ATQ protein consisting of the amino acid sequence represented by SEQ ID NO: 2, and has heavy chain CDR1, CDR2, and CDR3 consisting of the amino acid sequences represented by SEQ ID NOs: 4, 5, and 6 or 10, 11, and 12, respectively, and light chain CDR1, CDR2, and CDR3 consisting of the amino acid sequences represented by SEQ ID NOs: 7, 8, and 9 or 13, 14, and 15, respectively, or a binding fragment thereof.
(12) The kit according to (10), wherein the anti-APOA2-AT terminus antibody or the binding fragment thereof comprises an anti-APOA2-AT terminus monoclonal antibody that specifically binds to a carboxyl-terminal region of the APOA2-AT protein consisting of the amino acid sequence represented by SEQ ID NO: 1, and has heavy chain CDR1, CDR2, and CDR3 consisting of the amino acid sequences represented by SEQ ID NOs: 33, 34, and 35, SEQ ID NOs: 39, 40, and 41, or SEQ ID NOs: 45, 46, and 47, respectively, and light chain CDR1, CDR2, and CDR3 consisting of the amino acid sequences represented by SEQ ID NOs: 36, 37, and 38, SEQ ID NOs: 42, 43, and 44, or SEQ ID NOs: 48, 49, and 50, respectively, or a binding fragment thereof.
(13) The kit according to any of (10) to (12), further comprising an anti-APOA2 non-terminus monoclonal antibody that specifically binds to an amino acid sequence other than the carboxyl-terminal region of the APOA2-AT protein consisting of the amino acid sequence represented by SEQ ID NO: 1 or the APOA2-ATQ protein consisting of the amino acid sequence represented by SEQ ID NO: 2, and has heavy chain CDR1, CDR2, and CDR3 consisting of the amino acid sequences represented by SEQ ID NOs: 16, 17, and 18 or 22, 23, and 24, respectively, and light chain CDR1, CDR2, and CDR3 consisting of the amino acid sequences represented by SEQ ID NOs: 19, 20, and 21 or 25, 26, and 27, respectively.
(14) An anti-APOA2-AT terminus monoclonal antibody that specifically binds to a carboxyl-terminal region of the APOA2-AT protein consisting of the amino acid sequence represented by SEQ ID NO: 1, and has heavy chain CDR1, CDR2, and CDR3 consisting of the amino acid sequences represented by SEQ ID NOs: 33, 34, and 35, SEQ ID NOs: 39, 40, and 41, or SEQ ID NOs: 45, 46, and 47, respectively, and light chain CDR1, CDR2, and CDR3 consisting of the amino acid sequences represented by SEQ ID NOs: 36, 37, and 38, SEQ ID NOs: 42, 43, and 44, or SEQ ID NOs: 48, 49, and 50, respectively, or a binding fragment thereof.

The present specification encompasses the contents disclosed in Japanese Patent Application No. 2021-049931, on which the priority of the present application is based.

### Advantageous Effects of Invention

The present invention provides a convenient and noninvasive method that can assist in the determination of a test subject having pancreatic cystic tumor to have benign pancreatic cystic tumor or to have malignant pancreatic cystic tumor by merely measuring the concentrations of APOA2 protein variants comprised in a body fluid sample obtained from the test subject.

### Brief Description of Drawings

[Figure 1] Figure 1 shows results of measuring the binding activity of an anti-APOA2-AT terminus monoclonal antibody clones 4C6-1, 5D9-3, and 6B4-2 against various APOA2 protein variants.
[Figure 2] Figure 2 shows results of measuring APOA2-AT protein by sandwich ELISA using each of three anti-APOA2-AT terminus monoclonal antibodies (4C6-1, 5D9-3, or 6B4-2) or an anti-APOA2-AT terminus polyclonal antibody and an anti-APOA2 non-terminus monoclonal antibody.
[Figure 3] Figure 3 shows results of measuring and plotting the concentration of a pancreatic tumor marker CA19-9 comprised in the plasma of 15 cases of benign intraductal papillary mucinous neoplasm (IPMN) and 7 cases of malignant IPMN.
[Figure 4] Figure 4 shows results of measuring and plotting the concentration of APOA2-AT protein comprised in the plasma of 15 cases of benign IPMN and 7 cases of malignant IPMN by sandwich ELISA using a polyclonal antibody specifically recognizing the amino acid sequence of a carboxyl (C)-terminal region of the APOA2-AT protein (anti-APOA2-AT terminus polyclonal antibody) and an antibody specifically recognizing an amino acid sequence other than the C-terminal region (anti-APOA2 protein non-terminus antibody).
[Figure 5] Figure 5 shows results of measuring and plotting the concentration of APOA2-ATQ protein comprised in the plasma of 15 cases of benign IPMN and 7 cases of malignant IPMN by sandwich ELISA using a monoclonal antibody specifically recognizing the amino acid sequence of a C-terminal region of the APOA2-ATQ protein (anti-APOA2-ATQ terminus monoclonal antibody) and an antibody specifically recognizing an amino acid sequence other than the C-terminal region (anti-APOA2 protein non-terminus antibody).

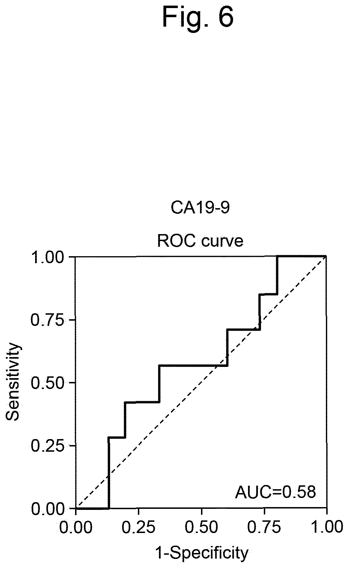

[Figure 6] Figure 6 shows a ROC curve showing the discrimination performance of 15 cases of benign IPMN and 7 cases of malignant IPMN using a pancreatic tumor marker CA19-9.

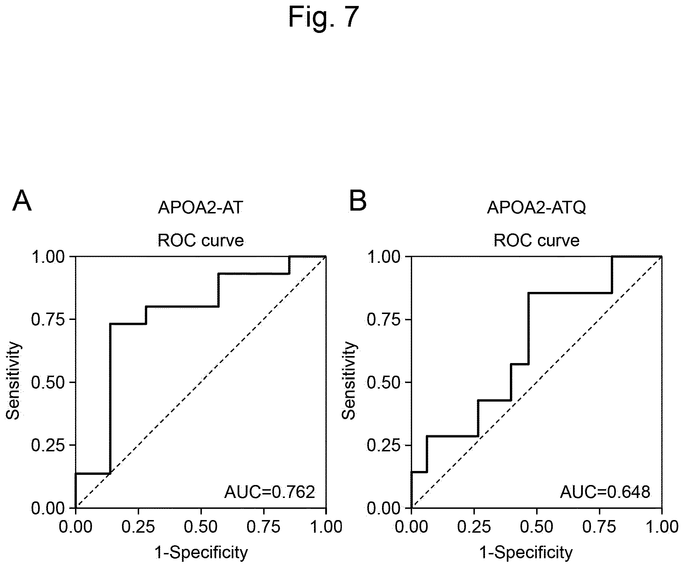

[Figure 7] Figure 7(A) shows a ROC curve showing the discrimination performance of 15 cases of benign IPMN and 7 cases of malignant IPMN using APOA2-AT protein. Figure 7(B) shows a ROC curve showing the discrimination performance of 15 cases of benign IPMN and 7 cases of malignant IPMN using APOA2-ATQ protein.

### Description of Embodiments

The target to be assayed according to the present invention is a test subject having pancreatic cystic tumor. In the present specification, the "pancreatic cystic tumor" refers to every tumor that is formed in the pancreas and exhibits the form of cyst retaining a liquid or mucus. Specifically, intraductal papillary mucinous neoplasm (IPMN), mucinous cystic neoplasm (MCN), and serous cyst neoplasm (SCN) are included therein.

In the present specification, the "malignant pancreatic cystic tumor" refers to a tumor with poor prognosis derived from pancreatic cystic tumor, and can be determined from HGD (high-grade dysplasia), the presence of an invasive cancer, or the presence of mural nodule. In the present specification, the "malignant pancreatic cystic tumor" also includes a common type of invasive pancreatic ductal adenocarcinoma (concomitant cancer) that is formed in the pancreas of a pancreatic cystic tumor patient and formed at a site different from pancreatic cystic tumor.

The effectiveness of a tumor marker (CA19-9) in blood has been reported for the prediction of such malignant pancreatic cystic tumor. However, work-up such as pancreatic juice cytology under ERCP or endoscopic ultrasound-guided fine needle aspiration (EUS-FNA) is required for definitive diagnosis (Non Patent Literature 1). When the "malignant pancreatic cystic tumor" is determined, treatment by resection appropriate for the site or spread of a lesion is discussed.

In the present specification, the "benign pancreatic cystic tumor" is a tumor that is low-grade dysplasia and is confined to a local area where the tumor has developed without invasion to its neighboring tissues or with invasion only to a limited local area.

### 1. Method for assisting in determination of malignant pancreatic cystic tumor using anti-APOA2 protein terminus antibody

The method of the present invention aims to detect malignant pancreatic cystic tumor in a test subject. The method of the present invention is a method for assisting in the determination of malignant pancreatic cystic tumor in a test subject having pancreatic cystic tumor, the method comprising the steps of measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, present in a body fluid sample obtained from the test subject, and assisting in the determination of the pancreatic cystic tumor of the test subject to be benign or malignant on the basis of the amount. An approach for measuring the APOA2-AT protein or/and the APOA2-ATQ protein is not particularly limited. A first embodiment of the method of the present invention is a method using an anti-APOA2 protein terminus antibody in the measurement of the APOA2-AT protein or/and the APOA2-ATQ protein. Hereinafter, the anti-APOA2 protein terminus antibody that may be used in the first embodiment of the present invention will be described.

### 1-1. Anti-APOA2 protein terminus antibody

In the present specification, the "APOA2 protein" corresponds to an APOA2 protein of each organism species and is preferably a human-derived APOA2 protein (GenBank Accession No. NP_001634.1), specifically includes human-derived wild-type APOA2 protein variants shown in SEQ ID NOs: 1, 2, and 3 and further includes their natural mutants and fragments thereof. In the present specification, the APOA2 protein refers to a protein whose carboxyl-terminal 6 amino acids have an amino acid sequence represented by any of SEQ ID NOs: 30 to 32.

In the present specification, the "variants" mean different molecular forms of the APOA2 protein that may be present in the plasma, serum, or other body fluid samples of humans or animals. The APOA2 protein variants correspond to, for example, APOA2 proteins differing in the structure of a C-terminal region, or their natural mutants. Specifically, the APOA2 protein variants correspond to, for example, APOA2-AT protein that is shown in SEQ ID NO: 1 and has the amino acid sequence of a C-terminal region ending in AT, APOA2-ATQ protein that is shown in SEQ ID NO: 2 and the amino acid sequence of a C-terminal region ending in ATQ, and APOA2-A protein that is shown in SEQ ID NO: 3 and the amino acid sequence of a C-terminal region ending in A.

In the present specification, the "carboxyl-terminal region (in the present specification, often referred to as the "C-terminal region")" refers to a region consisting of 6 to 25 amino acids, preferably 8 to 20 amino acids or 10 to 17 amino acids, including an amino acid at the carboxyl terminus (C terminus) and a few consecutive amino acids adjacent thereto in the amino acid sequence. In the present invention, the C-terminal region specifically refers to a region comprising an amino acid sequence of any of SEQ ID NOs: 30 to 32.

In the present specification, the "natural mutant" refers to a naturally occurring mutant having, for example, an amino acid sequence derived from the amino acid sequence represented by SEQ ID NO: 1, 2, or 3 by the deletion, substitution, or addition of one or several amino acids, or having 90% or higher, 92% or higher, or 94% or higher, preferably 95% or higher, more preferably 97% or higher, further preferably 98% or higher or 99% or higher identity to the amino acid sequence. The "identity" refers to the ratio (%) of the number of identical amino acid residues in one amino acid sequence to the number of all amino acid residues (including the number of gaps) in another amino acid sequence when these two amino acid sequences are aligned with or without gaps so as to attain the largest degree of coincidence. The term "several" refers to an integer of 2 to 10, for example, an integer of 2 to 7, 2 to 5, 2 to 4, or 2 or 3. Specific examples of the natural mutant include mutants based on polymorphisms such as SNPs (single nucleotide polymorphisms), and splicing mutants (splicing variants). The substitution is preferably conservative amino acid substitution. This is because the conservative amino acid substitution allows the resulting protein to have a structure or properties substantially equivalent to the APOA2 protein having the amino acid sequence described above. The conservative amino acids refer to the relationship among amino acids classified into the same amino acid groups. For example, a nonpolar amino acid group (glycine, alanine, phenylalanine, valine, leucine, isoleucine, methionine, proline, and tryptophan), a polar amino acid group (amino acids except for the nonpolar amino acids), a charged amino acid group (acidic amino acids (aspartic acid and glutamic acid) and a basic amino acid group (arginine, histidine, and lysine)), an uncharged amino acid group (amino acids except for the charged amino acids), an aromatic amino acid group (phenylalanine, tryptophan, and tyrosine), a branched amino acid group (leucine, isoleucine, and valine), and an aliphatic amino acid group (glycine, alanine, leucine, isoleucine, and valine) are known as the amino acid groups.

The "fragments thereof" refer to fragments of various APOA2 protein variants and their natural mutants, comprising the C-terminal regions of the APOA2 protein variants and the mutants. Specifically, the fragments thereof correspond to protease digestion products of various APOA2 protein variants and their mutants.

The method of the present invention can employ anti-APOA2 protein terminus antibodies including an anti-APOA2-AT terminus antibody or/and an anti-APOA2-ATQ terminus antibody. The method of the present invention can employ an anti-APOA2 protein non-terminus that does not bind to the C terminus of APOA2, in addition to the anti-APOA2 protein terminus antibody.

The "anti-APOA2-AT terminus antibody" refers to an antibody capable of specifically recognizing and binding to an epitope present in the C-terminal region of the APOA2-AT protein, specifically, a region comprising the amino acid sequence represented by SEQ ID NO: 30, or a binding fragment thereof. The phrase "specifically recognizing and binding" means that the antibody has no or very weak cross-reactivity with the other APOA2 protein variants and can thus neither recognize nor bind to or hardly recognizes and binds to the other APOA2 protein variants. Specifically, the anti-APOA2-AT terminus antibody refers to an antibody that specifically binds to the C-terminal region of the APOA2-AT protein, but exhibits no binding to the C-terminal region of the APOA2-ATQ protein and the C-terminal region of the APOA2-A protein. On the other hand, the "anti-APOA2-ATQ terminus antibody" refers to an antibody capable of specifically recognizing and binding to an epitope present in the C-terminal region of the APOA2-ATQ protein, specifically, a region comprising the amino acid sequence represented by SEQ ID NO: 31, or a fragment thereof. Specifically, the anti-APOA2-ATQ terminus antibody refers to an antibody that specifically binds to the C-terminal region of the APOA2-ATQ protein, but exhibits no binding to the C-terminal region of the APOA2-AT protein and the C-terminal region of the APOA2-A protein. Such an antibody directed to the terminus may be any of polyclonal and monoclonal antibodies or binding fragments thereof. A monoclonal antibody is preferred for achieving large-scale production and for obtaining homogeneous effects.

The "anti-APOA2 protein non-terminus antibody" refers to an anti-APOA2 antibody recognizing and binding to an epitope present in a region other than the C-terminal region in the full-length amino acid sequence of each APOA2 protein variant. Specifically, the anti-APOA2 protein non-terminus antibody totally differs from the anti-APOA2 protein terminus antibodies in epitope recognized thereby. The term "non-terminus" for the anti-APOA2 protein non-terminus antibody is used for the sake of convenience with respect to the anti-APOA2 protein terminus antibodies. Thus, its epitope is not particularly limited as long as the epitope is present in a region other than the C-terminal region. The anti-APOA2 protein non-terminus antibody can also include an antibody recognizing and binding to an epitope present in the N terminus. In the present specification, antibodies simply referred to as "anti-APOA2 antibodies" refer to antibodies including both an anti-APOA2 protein terminus antibody and an anti-APOA2 protein non-terminus antibody.

The method of the present invention can employ an anti-APOA2 protein non-terminus antibody. The anti-APOA2 protein non-terminus antibody is preferably an antibody that has almost the same levels of binding activity against an APOA2 protein (e.g., APOA2-AT) having a certain C-terminal sequence and binding activity against an APOA2 protein (e.g., APOA2-ATQ) having a C-terminal sequence different therefrom, and does not inhibit the binding of the anti-APOA2 protein terminus antibodies to the C-terminal regions. Specific examples thereof include an "anti-APOA2-AT non-terminus antibody" binding to an amino acid sequence other than the C-terminal region of the APOA2-AT protein shown in SEQ ID NO: 1, an "anti-APOA2-ATQ non-terminus antibody" binding to an amino acid sequence other than the C-terminal region of the APOA2-ATQ protein shown in SEQ ID NO: 2. In this case, the antibodies have the same levels of binding activity against these APOA2 proteins, and any of the antibodies do not inhibit the binding of the anti-APOA2-AT terminus antibody and the anti-APOA2-ATQ terminus antibody to the C-terminal regions of the APOA2 proteins. The anti-APOA2 protein non-terminus antibody may be any of polyclonal and monoclonal antibodies or binding fragments thereof. A monoclonal antibody is preferred for achieving large-scale production and for obtaining homogeneous effects.

The "monoclonal antibody" used in the present specification refers to an antibody that comprises a single immunoglobulin or framework regions (hereinafter, referred to as "FRs") and complementarity determining regions (hereinafter, referred to as "CDRs") and is capable of specifically recognizing and binding to a particular antigen (epitope).

The typical immunoglobulin molecule is a tetramer constituted by two polypeptide chain pairs, i.e., two heavy-light chain pairs, in which the heavy chain in each pair is linked to its partner light chain through a disulfide bond. Each heavy chain is composed of a heavy chain variable region (H chain V region; hereinafter, referred to as "VH") on the N-terminal side and a heavy chain constant region (H chain C region; hereinafter, referred to as "CH") on the C-terminal side. Each light chain is composed of a light chain variable region (L chain V region; hereinafter, referred to as "VL") on the N-terminal side and a light chain constant region (L chain C region; hereinafter, referred to as "CL") on the C-terminal side. Of these regions, VH and VL are particularly important because of their involvement in the binding specificity of the antibody. These VH and VL regions each consist of approximately 110 amino acid residues and internally have three CDRs (CDR1, CDR2, and CDR3) involved directly in the binding specificity for the antigen and four FRs (FR1, FR2, FR3, and FR4) functioning as the backbone structures of the variable region. The CDRs are known to be conformationally complementary to the antigen molecule and to determine the specificity of the antibody (E.A. Kabat et al., 1991, Sequences of proteins of immunological interest, Vol. 1, eds. 5, NIH publication). The amino acid sequences of the constant regions rarely vary among intraspecific antibodies, whereas the amino acid sequences of the CDRs are highly variable among antibodies and, hence, are also called hypervariable regions. In the variable region, the CDRs and the FRs are arranged in the order of FR1, CDR1, FR2, CDR2, FR3, CDR3, and FR4 from the N terminus toward the C terminus. In the immunoglobulin molecule, VL and VH are paired by dimerization to form an antigen-binding site. The immunoglobulin is known to have each class of IgG, IgM, IgA, IgE, and IgD. The antibody for use in the method of the present invention may be of any class. IgG is preferred.

The anti-APOA2-ATQ terminus monoclonal antibody of the present invention specifically binds to the C-terminal region of the APOA2-ATQ protein shown in SEQ ID NO: 2, but exhibits no binding activity against the APOA2-AT protein shown in SEQ ID NO: 1 and the APOA2-A protein shown in SEQ ID NO: 3. Specific examples of such an antibody include anti-APOA2-ATQ terminus monoclonal antibody clones represented by antibody clone names 7F2 and 6G2. The clone 7F2 has CDR1 consisting of the sequence represented by SEQ ID NO: 4, CDR2 consisting of the sequence represented by SEQ ID NO: 5, and CDR3 consisting of the sequence represented by SEQ ID NO: 6 in a heavy chain, and CDR1 consisting of the sequence represented by SEQ ID NO: 7, CDR2 consisting of the sequence represented by SEQ ID NO: 8, and CDR3 consisting of the sequence represented by SEQ ID NO: 9 in a light chain. The clone 6G2 has CDR1 consisting of the sequence represented by SEQ ID NO: 10, CDR2 consisting of the sequence represented by SEQ ID NO: 11, and CDR3 consisting of the sequence represented by SEQ ID NO: 12 in a heavy chain, and CDR1 consisting of the sequence represented by SEQ ID NO: 13, CDR2 consisting of the sequence represented by SEQ ID NO: 14, and CDR3 consisting of the sequence represented by SEQ ID NO: 15 in a light chain.

The anti-APOA2-AT terminus monoclonal antibody of the present invention specifically binds to the C-terminal region of the APOA2-AT protein shown in SEQ ID NO: 1, but exhibits no binding activity against the APOA2-ATQ protein shown in SEQ ID NO: 2 and the APOA2-A protein shown in SEQ ID NO: 3. Specific examples of such an antibody include anti-APOA2-AT terminus monoclonal antibody clones represented by antibody clone names 4C6-1, 5D9-3, and 6B4-2. The clone 4C6-1 has CDR1 consisting of the sequence represented by SEQ ID NO: 33, CDR2 consisting of the sequence represented by SEQ ID NO: 34, and CDR3 consisting of the sequence represented by SEQ ID NO: 35 in a heavy chain, and CDR1 consisting of the sequence represented by SEQ ID NO: 36, CDR2 consisting of the sequence represented by SEQ ID NO: 37, and CDR3 consisting of the sequence represented by SEQ ID NO: 38 in a light chain. The clone 5D9-3 has CDR1 consisting of the sequence represented by SEQ ID NO: 39, CDR2 consisting of the sequence represented by SEQ ID NO: 40, and CDR3 consisting of the sequence represented by SEQ ID NO: 41 in a heavy chain, and CDR1 consisting of the sequence represented by SEQ ID NO: 42, CDR2 consisting of the sequence represented by SEQ ID NO: 43, and CDR3 consisting of the sequence represented by SEQ ID NO: 44 in a light chain. The clone 6B4-2 has CDR1 consisting of the sequence represented by SEQ ID NO: 45, CDR2 consisting of the sequence represented by SEQ ID NO: 46, and CDR3 consisting of the sequence represented by SEQ ID NO: 47 in a heavy chain, and CDR1 consisting of the sequence represented by SEQ ID NO: 48, CDR2 consisting of the sequence represented by SEQ ID NO: 49, and CDR3 consisting of the sequence represented by SEQ ID NO: 50 in a light chain.

The anti-APOA2 protein non-terminus antibody of the present invention is preferably an antibody having the same levels of binding activity against the APOA2 protein variants shown in SEQ ID NOs: 1 to 3 when the binding activity is compared among them. Specific examples thereof include anti-APOA2 antibody clones represented by antibody clone names MAB 1 and MAB2. The clone MAB1 has CDR1 consisting of the amino acid sequence represented by SEQ ID NO: 16, CDR2 consisting of the amino acid sequence represented by SEQ ID NO: 17, and CDR3 consisting of the amino acid sequence represented by SEQ ID NO: 18 in a heavy chain, and CDR1 consisting of the amino acid sequence represented by SEQ ID NO: 19, CDR2 consisting of the amino acid sequence represented by SEQ ID NO: 20, and CDR3 consisting of the amino acid sequence represented by SEQ ID NO: 21 in a light chain. The clone MAB2 has CDR1 consisting of the amino acid sequence represented by SEQ ID NO: 22, CDR2 consisting of the amino acid sequence represented by SEQ ID NO: 23, and CDR3 consisting of the amino acid sequence represented by SEQ ID NO: 24 in a heavy chain, and CDR1 consisting of the amino acid sequence represented by SEQ ID NO: 25, CDR2 consisting of the amino acid sequence represented by SEQ ID NO: 26, and CDR3 consisting of the amino acid sequence represented by SEQ ID NO: 27 in a light chain. The anti-APOA2-ATQ non-terminus antibody or the anti-APOA2-AT non-terminus antibody can be used as the anti-APOA2 protein non-terminus antibody.

The "binding fragments thereof" for the "polyclonal and monoclonal antibodies or binding fragments thereof" are partial fragments of the polyclonal and monoclonal antibodies having an epitope in a C-terminal region of the APOA2 protein or a region other than the C terminus, and are polypeptide chains having activity substantially equivalent to the antigen-specific binding activity of the antibodies, or complexes thereof. The binding fragments each correspond to an antibody portion comprising at least one antigen-binding site, i.e., a polypeptide chain having at least one VL-VH pair, or a complex thereof. Specific examples thereof include a large number of sufficiently characterized antibody fragments resulting from the cleavage of an immunoglobulin with various peptidases. More specific examples thereof include Fab, F(ab')₂, and Fab'. The Fab is a fragment resulting from the papain cleavage of the IgG molecule on the N-terminal side of the disulfide bonds in the hinges and is constituted by a polypeptide consisting of VH and CH1, which is adjacent to the VH, among the three CH-constituting domains (CH1, CH2, and CH3), and a light chain. The F(ab')₂ is a Fab' dimer resulting from the pepsin cleavage of the IgG molecule on the C-terminal side of the disulfide bonds in the hinges. The Fab' is substantially structurally equivalent to Fab, though being slightly longer at H chain than Fab by including a hinge (Fundamental Immunology, Paul ed., 3rd ed., 1993). The Fab' can be obtained by reducing F(ab')₂ under mild conditions and cleaving the disulfide bridges in the hinge region. All of these antibody fragments comprise the antigen-binding site and have the ability to specifically bind to the antigen (i.e., a particular APOA2 protein variant in the present invention).

The binding fragment of the antibody for use in the method of the present invention may be synthesized chemically or by use of a recombinant DNA method. Examples thereof include antibody fragments newly synthesized using the recombinant DNA method. Specifically, the fragment corresponds to, but is not limited to, a monomeric polypeptide molecule in which one or more VLs and one or more VHs of the antibody for use in the method of the present invention are artificially linked via a linker peptide or the like having an appropriate length of a sequence, or a multimeric polypeptide thereof. Examples of such a polypeptide include synthetic antibodies such as single-chain Fv (scFv: single chain fragment of variable region) (see Pierce catalog and Handbook, 1994-1995, Pierce Chemical co., Rockford, IL), diabody, triabody, and tetrabody. In the immunoglobulin molecule, VL and VH are normally positioned on separate polypeptide chains (L chain and H chain). The single-chain Fv is a synthetic antibody fragment having a structure where these variable regions are linked via a flexible linker having a sufficient length such that the VL and the VH are comprised in one polypeptide chain. Both of the variable regions in the single-chain Fv are self-assembled with each other to form one functional antigen-binding site. The single-chain Fv can be obtained by integrating a recombinant DNA encoding the single-chain Fv into the phage genome using a technique known in the art, followed by expression. The diabody is a molecule having a structure based on the dimeric structure of the single-chain Fvs (Holliger et al., 1993, Proc. Natl. Acad. Sci USA, 90: 6444-6448). For example, when the linker has a length shorter than approximately 12 amino acid residues, the two variable sites in the single-chain Fv cannot be self-assembled. By contrast, VL in one Fv chain can be assembled with VH in another Fv chain by the formation of the diabody, i.e., by the interaction between the two single-chain Fvs. As a result, two functional antigen-binding sites can be formed (Marvin et al., 2005, Acta Pharmacol. Sin., 26: 649-658). The further addition of cysteine residues to the C termini of the single-chain Fvs permits a disulfide bond between these two Fv chains so that stable diabody can be formed (Alafsen et al., 2004, Prot. Engr. Des. Sel., 17: 21-27). Although the diabody is a divalent antibody fragment as described above, its antigen-binding sites do not have to bind to the same epitope and may have bispecificity of recognizing and specifically binding to different epitopes, respectively. The triabody or the tetrabody has a trimeric or tetrameric structure based on the single-chain Fv structure, as in the diabody. The triabody and the tetrabody are trivalent and quadrivalent antibody fragments, respectively, and may each be a multispecific antibody. The antibody fragment for use in the method of the present invention further includes antibody fragments identified using a phage display library (see e.g., McCafferty et al., 1990, Nature, Vol. 348, 522-554), wherein these antibody fragments have the ability to bind to their antigens. Also see, for example, Kuby, J., Immunology, 3rd Ed., 1998, W.H. Freeman & Co., New York.

In the present invention, each anti-APOA2 antibody can be modified. In this context, the modification includes any of functional modifications (e.g., glycosylation) required for the anti-APOA2-ATQ terminus antibody to have specific binding activity against the particular APOA2 protein variant, and labeling necessary for detecting the antibody for use in the method of the present invention. Examples of the antibody labeling include labeling with fluorescent dyes (FITC, rhodamine, Texas Red, Cy3, and Cy5), fluorescent proteins (e.g., PE, APC, and GFP), enzymes (e.g., horseradish peroxidase, alkaline phosphatase, and glucose oxidase), or biotin or (strept)avidin. The antibody glycosylation may be altered in order to adjust the affinity of the antibody for the antigen. Such alteration can be achieved, for example, by changing one or more glycosylation sites in the antibody sequence. To be more specific, one or more amino acid substitutions can be introduced to an amino acid sequence constituting one or more glycosylation sites, for example, in FR, to remove the glycosylation sites. As a result, the glycosylation of the sites can be canceled. Such deglycosylation is effective for increasing the affinity of the antibody for the antigen (U.S. Patent Nos. 5714350 and 6350861).

### 1-2. Preparation of immunogen

In preparing the anti-APOA2 protein terminus antibodies in the present invention, each APOA2 protein variant is prepared as an immunogen (antigen). Examples of the APOA2 protein variant that can be used as an immunogen in the present invention include APOA2 proteins having the amino acid sequence represented by SEQ ID NOs: 1 to 3 and mutants thereof, and polypeptide fragments of the proteins or the mutants, and their fusion polypeptides with other peptides (e.g., signal peptides and labeling peptides). The APOA2 protein variant as an immunogen can be synthesized by an approach known in the art, for example, a solid-phase peptide synthesis method, for example, using information on the amino acid sequence represented by SEQ ID NOs: 1 to 3. The APOA2 protein variant can be prepared by, for example, a method given below.

Any of naturally occurring APOA2 proteins, recombinant APOA2 proteins, and synthetic APOA2 proteins, the whole or a portion of which has been chemically synthesized can be used as the APOA2 protein variant. For example, a variant derived from any of naturally occurring APOA2 proteins, recombinant APOA2 proteins, and synthetic APOA2 proteins can be used as an antigenic APOA2 protein variant that is prepared in order to obtain each antibody binding to the APOA2 protein C terminus (anti-APOA2 protein terminus antibody) as long as the variant comprises an amino acid sequence consisting of 6 or more consecutive amino acids of the C-terminal region.

The naturally occurring APOA2 proteins can be recovered from samples including body fluid samples such as blood (including plasma and serum), or culture supernatants of cultured cells by use of a protein separation and purification technique known in the art, for example, gel filtration, ion-exchange chromatography, or affinity chromatography.

The recombinant APOA2 proteins can be expressed in microbes, insect cells, or animal cells harboring DNAs encoding the proteins and then recovered from the cells by use of a protein separation and purification technique known in the art.

The synthetic APOA2 proteins can be synthesized by an approach known in the art, for example, a solid-phase peptide synthesis method, for example, using published information on the amino acid sequence of the APOA2 protein. These synthetic APOA2 proteins may each be linked to a carrier protein such as KLH (keyhole limpet hemocyanin), OVA (ovalbumin), or BSA (bovine serum albumin).

In the case of using a fragment of the APOA2 protein variant as an immunogen in the anti-APOA2 protein terminus antibody preparation, any of naturally occurring APOA2 protein fragments, recombinant APOA2 protein fragments, and synthetic APOA2 protein fragment may also be used. For example, an oligopeptide or a polypeptide comprising 6 or more, preferably 10 or more, more preferably 18 or more, further preferably 30 or more consecutive amino acid residues including the C terminus of an amino acid sequence represented by any of SEQ ID NOs: 1 to 3 can be used as the APOA2 protein fragment serving as an antigen. For example, a peptide comprising the amino acid sequences represented by SEQ ID NO: 28 or 29 can be used.

In the case of using a fragment of the naturally occurring APOA2 protein as an immunogen, for example, in the anti-APOA2 protein terminus antibody preparation, the purified APOA2 protein is first treated with suitable protease such as trypsin and then fractionated on a reverse-phase column to obtain peaks. Subsequently, the amino acid sequence of the peptide comprised in each peak is determined with a mass spectrometer. The peptide, when comprising, as a partial sequence, a sequence consisting of 6 or more consecutive amino acids of the C-terminal region of the APOA2 protein shown in any of SEQ ID NOs: 1 to 3, can be used as the immunogen.

In the case of using a partial amino acid sequence of the recombinant APOA2 protein as an immunogen, for example, in the anti-APOA2 protein terminus antibody preparation, a DNA sequence encoding a peptide (C-terminal fragment) consisting of a partial sequence of 6 or more consecutive amino acids including the C-terminal amino acid residues of the APOA2 protein shown in any of SEQ ID NOs: 1 to 3 is first inserted to vectors for expressions. Next, the vectors for expressions are transferred to various cells to express the encoded C-terminal fragment. Finally, the C-terminal fragment is extracted from the cells after the expression according to a routine method. The obtained C-terminal fragment can be used as the immunogen.

Also in the case of preparing the anti-APOA2 protein non-terminus antibody in the present invention, its preparation method can be basically the same as the method for preparing the anti-APOA2 protein terminus antibodies except that a region that can be used as an immunogen in the APOA2 protein is different from the regions used as an immunogen for preparing the anti-APOA2 protein terminus antibodies. Specifically, the whole or a portion of a region other than the C-terminal region of the APOA2 protein can be used as an immunogen. In the case of preparing the anti-APOA2 protein non-terminus antibody, as in the case of preparing the anti-APOA2 protein terminus antibodies, an oligopeptide or a polypeptide comprising amino acid residues of the region other than the C-terminal region of the APOA2 protein can also be used as an antigen.

### (Preparation of recombinant APOA2 protein)

Hereinafter, the preparation of a recombinant APOA2 protein (recombinant APOA2 protein variant) shown in any of SEQ ID NOs: 1 to 3 will be described in detail.

### (a) Preparation of polynucleotide encoding recombinant APOA2 protein variant

Phages or plasmids capable of autonomously replicating in host microbes can be used as vectors for use in the expression of various APOA2 protein variants. Examples of the plasmids include *E*. *coli*-derived plasmids (pET30a, pGEX6p, pUC118, pUC119, pUC18, pUC19, etc.), *Bacillus subtilis*-derived plasmids (pUB110, pTP5, etc.), and enzyme-derived plasmids (YEp13, YEp24, YCp50, etc.). Examples of the phages include λ phages (λgt11, λZAP, etc.). In addition, vectors of animal viruses such as vaccinia virus or insect viruses such as baculovirus can also be used.

The method for inserting a polynucleotide encoding the APOA2 protein variant to the vectors involves, for example, cleaving the purified polynucleotide with corresponding appropriate restriction enzymes and ligating the resulting fragment into the vectors cleaved with the appropriate restriction enzymes by use of DNA ligase or the like.

### (b) Transfer of APOA2 protein variant expression vector into host

The obtained APOA2 protein variant expression vectors are transferred to hosts capable of expressing the expression vectors to obtain transformants capable of expressing the APOA2 protein variant (variant-expressing transformants). The hosts used are not particularly limited as long as the hosts are suitable for the vectors used and are capable of expressing the APOA2 protein variant. For example, bacteria (*E. coli* (*Escherichia coli*), *Bacillus subtilis*, yeasts, insect cells, or animal cells (COS cells and CHO cells (Journal of immunology, 1998, Vol. 160, 3393-3402)) are preferably used. The method for transferring the vectors to the bacteria is not particularly limited as long as the method is a method known in the art for transferring the vectors to the bacteria. Examples thereof include a heat shock method, a method using calcium ions, and electroporation. All of these techniques are known in the art and described in various literatures. See, for example, Greene & Sambrook, 2012, Molecular Cloning: A Laboratory Manual Fourth Ed., Cold Spring Harbor Laboratory Press, Cold Spring Harbor, New York. A Lipofectin method (PNAS, 1989, Vol. 86, 6077; and PNAS, 1987, Vol. 84, 7413), electroporation, a calcium phosphate method (Virology, 1973, Vol. 52, 456-467), a DEAE-dextran method or the like is preferably used in the transformation of the animal cells.

In the case of using bacteria as the hosts, preferably, the APOA2 protein variant expression vectors are capable of autonomously replicating in the bacteria and are also constituted by a promoter sequence, a ribosomal binding sequence, the DNA sequence encoding the APOA2 protein variant, and a transcription termination sequence. The expression vectors may also comprise a gene encoding a regulatory factor controlling the promoter. Any promoter that can function in the hosts such as *E. coli* may be used.

Likewise, in the case of using eukaryotic cells such as yeasts, animal cells, or insect cells as the hosts, APOA2 protein variant-expressing transformants can also be obtained according to an approach known in the art. The APOA2 protein variant expression vectors for use in the eukaryotic cells comprise a promoter sequence and the DNA sequence encoding the APOA2 protein variant, which may be linked, if desired, to a cis element (e.g., an enhancer), a splicing signal (a donor site, an acceptor site, a branch point, etc.), a poly-A addition signal, a selective marker sequence, a ribosomal binding sequence (SD sequence), and the like.

### (c) Culture of variant-expressing transformant and expression of recombinant APOA2 protein variant

Subsequently, the prepared variant-expressing transformants are cultured. The method for culturing the variant-expressing transformants in a medium is carried out according to an ordinary method for use in the culture of the hosts. In the case of using, for example, bacteria as the hosts, the medium is not particularly limited as long as the medium comprises a carbon source, a nitrogen source, inorganic salts, etc., utilizable by the bacteria and the bacteria are capable of growing or proliferating in the medium. Any of natural and synthetic media can be used. More specific examples thereof include an LB medium, but are not limited thereto, as a matter of course. For the selective culture of the variant-expressing transformants, an antibiotic such as ampicillin or tetracycline may be added to the medium, if necessary. The culture is usually carried out at 37°C for 6 to 24 hours under aerobic conditions such as culture with aeration and stirring. During the culture period, the pH is preferably kept at or around a neutral pH. The pH is adjusted using an inorganic or organic acid or alkali solution or the like. When the variant-expressing transformants are animal cells such as CHO cells, the host cells can be inoculated at 1 × 10⁵ cells/mL to a DMEM medium manufactured by Life Technologies Corp. (currently known as Thermo Fisher Scientific Inc.) and cultured in a 5% CO₂ incubator of 37°C. During the culture, an antibiotic such as ampicillin or tetracycline may be added to the medium, if necessary.

When the APOA2 protein variant expression vectors are protein expression induction-type vectors comprising a protein expression control system (which corresponds to, for example, a repressor gene and an operator for the host bacteria), the expression of the APOA2 protein variant needs to be induced by a predetermined treatment of the variant-expressing transformants. The expression induction method differs depending on the protein expression control system comprised in the vectors. Therefore, induction treatment suitable for the system can be carried out. For example, the protein expression control system most generally used in the protein expression induction-type vectors for use in the host bacteria is a system consisting of a lac repressor gene and a lac operator. This system is capable of inducing the expression by IPTG (isopropyl-1-thio-β-D-galactoside) treatment. The transformants having the APOA2 protein expression vectors comprising this system can be allowed to express the APOA2 protein variant of interest by the addition of IPTG in an appropriate amount (e.g., final concentration: 1 mM) into the medium.

### (d) Extraction and/or recovery of recombinant APOA2 protein variant

When the APOA2 protein variant is produced inside the bacterial bodies or the cells after the culture, the bacterial bodies or the cells can be recovered and disrupted, followed by the extraction of the protein of interest. When the APOA2 protein variant is secreted outside the bacterial bodies or the cells, the culture solution can be used directly, or the supernatant obtained by the removal of the bacterial bodies or the cells through centrifugation or the like can be used. Then, the APOA2 protein variant can be isolated and purified from the cultures by using, alone or in appropriate combination, general protein purification methods, for example, ammonium sulfate precipitation, gel filtration, ion-exchange chromatography, and affinity chromatography. Whether or not the APOA2 protein variant has been obtained can be confirmed by SDS-polyacrylamide gel electrophoresis or the like.

### 1-3. Preparation of anti-APOA2 monoclonal antibody

### 1-3-1. Methods for preparing anti-APOA2 monoclonal antibody and hybridoma

Hybridomas producing the anti-APOA2 monoclonal antibody of the present invention can be prepared by a method described below. However, the preparation method is not limited thereto, and any of other methods known in the art can be used for the preparation.

### (1) Method for preparing anti-APOA2 monoclonal antibody

In order to prepare the anti-APOA2 protein terminus monoclonal antibody specifically binding to the C-terminal region of any APOA2 protein shown in SEQ ID NO: 1, 2, or 3 in the amino acid sequence constituting the APOA2 protein, monoclonal antibodies can be prepared with the APOA2 protein variant or a peptide comprising the C-terminal region of the APOA2 protein variant as an immunogen and then screened for an antibody binding only to the particular APOA2 protein variant using the APOA2 protein shown in any of SEQ ID NOs: 1 to 3 or the peptide comprising the C-terminal region of the APOA2 protein variant. For example, the anti-APOA2-ATQ terminus monoclonal antibody can be selected by screening using, as an index, specific binding to the C-terminal region (region comprising the amino acid sequence of SEQ ID NO: 31) of the APOA2-ATQ protein shown in SEQ ID NO: 2 without or almost without binding to the APOA2 protein variant shown in SEQ ID NO: 1 or 3. Also, the anti-APOA2-AT terminus monoclonal antibody can be selected by screening using, as an index, specific binding to the C-terminal region (region comprising the amino acid sequence of SEQ ID NO: 30) of the APOA2-AT protein shown in SEQ ID NO: 1 without or almost without binding to the APOA2 protein variant shown in SEQ ID NO: 2 or 3.

In order to prepare the anti-APOA2 protein non-terminus antibody recognizing amino acids other than the C-terminal region of the APOA2 protein, monoclonal antibodies can be prepared with the APOA2 protein variant or a peptide comprising a partial sequence thereof as an immunogen and then screened for the antibody of interest by using, as an index, the same levels of binding activity against the APOA2 protein variants shown in SEQ ID NOs: 1 to 3 or their peptides differing in C terminus when the binding activity is compared among them.

### (2) Preparation of anti-APOA2 protein terminus antibody-producing cell

The recombinant APOA2 protein obtained as an immunogen in the paragraph 1-2 is dissolved in a buffer solution to prepare an immunogen solution. For effective immunization, an adjuvant may be added thereto, if necessary. Examples of the adjuvant include commercially available Freund's complete adjuvant (FCA) and Freund's incomplete adjuvant (FIA). These adjuvants may be used alone or as a mixture.

Next, a mammal, for example, a rat, a mouse (e.g., an inbred mouse BALB/c), or a rabbit is immunized by the administration of the prepared immunogen solution. Examples of the immunogen administration method include, but are not limited to, subcutaneous injection using FIA or FCA, intraperitoneal injection using FIA, and intravenous injection using 0.15 M sodium chloride. One dose of the immunogen is appropriately determined according to the type of the animal to be immunized, an administration route, etc., and is approximately 50 to 200 µg per animal. The intervals between the immunization shots are not particularly limited. After the priming, 2 to 6, preferably 3 or 4 boosters are performed at intervals of a few days to a few weeks, preferably at 1- to 4-week intervals. After the priming, an antibody titer in the serum of the immunized animal is measured by ELISA (enzyme-linked immunosorbent assay) or the like. Provided that a sufficient rise in antibody titer is shown, the immunogen is intravenously or intraperitoneally injected for final immunization. 2 to 5 days, preferably 3 days, after the final immunization date, antibody-producing cells are collected.

### 1-3-2. Method for preparing anti-APOA2 monoclonal antibody-producing hybridoma

### (1) Recovery of antibody-producing cell from immunized animal and cell fusion

The antibody-producing cells obtained from the immunized animal can be subjected to cell fusion with myeloma cells to prepare hybridomas producing the monoclonal antibody specifically recognizing the particular region of the APOA2 protein. Examples of the antibody-producing cells include spleen cells, lymph node cells, and peripheral blood cells. Spleen cells or local lymph node cells are preferred. A generally available established cell line derived from mice or the like can be used as the myeloma cells for fusion with the antibody-producing cells. The cell line used preferably has drug selectivity and has the property of being unable to survive in an unfused state in a HAT selective medium (comprising hypoxanthine, aminopterin, and thymidine) and being able to grow therein only in a state fused with the antibody-producing cells. Also, the established cell line is preferably derived from an animal of the same species as the immunized animal. Specific examples of the myeloma cells include BALB/c mouse-derived hypoxanthine-guanine-phosphoribosyl-transferase (HGPRT)-deficient cell lines P3X62-Ag.8 (ATCCTIB9), P3X63-Ag.8.U1 (JCRB9085), P3/NSI/1-Ag4-1 (JCRB0009), P3x63Ag8.653 (JCRB0028), and SP2/0-Ag14 (JCRB0029).

For the cell fusion between the myeloma cells and the antibody-producing cells, the antibody-producing cells and the myeloma cells are mixed at a ratio of approximately 1:1 to 20:1 in a serum-free medium for animal cell culture, such as a DMEM or RPMI1640 medium, and fused with each other through reaction in the presence of a cell fusion promoter. For example, polyethylene glycol having an average molecular weight of 1,500 to 4,000 Da can be used as the cell fusion promoter at a concentration of approximately 10 to 80%. If necessary, an auxiliary such as dimethyl sulfoxide may be used in combination therewith for enhancing fusion efficiency. Alternatively, the antibody-producing cells and the myeloma cells may be fused with each other using a commercially available cell fusion apparatus that employs electric stimulation (e.g., electroporation) (Nature, 1977, Vol. 266, 550-552).

### (2) Selection of hybridoma of interest

Examples of the method for selecting hybridomas producing the anti-APOA2 monoclonal antibody of interest from the cells after the cell fusion treatment include the following method. The cell suspension is appropriately diluted with, for example, an RPMI1640 medium comprising fetal bovine serum and then seeded at approximately 2 × 10⁶ cells/well over a 96-well microtiter plate. A selective medium is added to each well where the cells are subsequently cultured with the selective medium appropriately replaced with a fresh one. The culture temperature is set to 20 to 40°C, preferably approximately 37°C. When the myeloma cells are of HGPRT-deficient line or thymidine kinase (TK)-deficient line, only hybridomas of the antibody-producing cells and the myeloma cells can be selectively allowed to grow or proliferate by use of a selective medium comprising hypoxanthine, aminopterin, and thymidine (HAT medium). Therefore, cells grown from approximately 10 days after the start of culture in the selective medium can be selected as the hybridomas.

Antibodies produced by the hybridomas selected in the HAT medium are first screened by using binding activity against various APOA2 protein variants shown in SEQ ID NOs: 1 to 3 as an index. Subsequently, the antibody having binding activity is tested for cross-reactivity to select acceptable ones. The "acceptable ones (cross-reactivity)" mean cross-reactivity at a negligible level for the intended purposes of the antibody. For example, a monoclonal antibody for use in immunological assay can be interpreted as having practically no cross-reactivity when signal intensity from cross reaction in a final assay system can be suppressed at a background level to less than 1% of signal intensity from specific reaction.

For example, ELISA can be used for confirming reaction specificity for the particular APOA2 protein variant. In this ELISA method, a microplate in which various APOA2 protein variants or fragments thereof are separately immobilized as antigens on different wells is prepared and reacted by the addition of appropriately diluted samples of the culture supernatant of the hybridomas. After sufficient reaction, the wells are washed and further reacted by the addition of a labeled form of a secondary antibody directed to an immunoglobulin. The wells are washed again and can be finally assayed by use of the label of the secondary antibody bound with the wells to quantitatively determine the binding activity of the antibody present in the culture supernatant against the antigens. For example, for the preparation of the anti-APOA2 protein terminus monoclonal antibody, the specificity can be judged by the presence of binding activity only against the C-terminal region of the particular APOA2 protein variant without cross-reactivity with the other APOA2 protein variants. For the preparation of the anti-APOA2 protein non-terminus monoclonal antibody, the antibody is selected by using, as an index, the same levels of binding activity against all of the APOA2 protein variants differing in C terminus without the inhibition of the binding of the anti-APOA2 protein terminus monoclonal antibody to the C-terminal region by the prepared antibody.

The hybridomas can also be selected by use of a recombinant DNA technique. First, mRNAs are extracted from the hybridoma group obtained according to the aforementioned method. The mRNA extraction can be carried out by use of a method known in the art. Subsequently, cDNAs are obtained from the mRNAs using Oligo dT primers or random primers. The cDNAs are used as templates in PCR using primer sets comprising the nucleotide sequence of the signal sequence upstream of the variable region-encoding gene and a nucleotide sequence on the constant region side. The obtained amplification products can be inserted to appropriate cloning vectors and cloned to obtain a library of the variable region genes of the antibodies produced by the hybridomas. As a more specific non-limiting example, PCR is carried out using Mouse Ig Primer provided by Merck Millipore, and the amplification products (mouse immunoglobulin variable region cDNAs) are inserted to the EcoRI sites of ZERO BLUNT PCR TOPO Vectors provided by Life Technologies Corp. (currently known as Thermo Fisher Scientific, Inc.) and cloned. The obtained vector group can be used as a library of the genes encoding the variable region amino acid sequences. Next, a probe is designed on the basis of the amino acid sequence of each variable region or each CDR disclosed in the present invention. The library can be screened for positive clones to select the hybridomas producing the antibody for use in the method of the present invention.

### (3) Antibody production using hybridoma

The hybridomas according to the present invention can be used in antibody production by ascites formation using a mouse. Specifically, the hybridomas are intraperitoneally inoculated to a mouse of the origin of the fusion partner cells used for preparing the hybridomas, or to a nude mouse. The ascites can be appropriately collected to recover an antibody-comprising ascites fluid. More specifically, hybridomas obtained with SP2/0 cells as a fusion partner are intraperitoneally inoculated to a BALB/c mouse 10 days after inoculation with pristine, and an antibody-comprising ascites fluid can be recovered.

The hybridomas according to the present invention can be used in antibody production by culture using a suitable medium. Specifically, the hybridomas can be inoculated at 1 × 10⁵ cells/mL into a Hybridoma-SFM medium manufactured by Life Technologies Corp. (currently known as Thermo Fisher Scientific, Inc.) and cultured in a 5% CO₂ incubator of 37°C until the hybridomas are killed to obtain an antibody-comprising culture supernatant, though the antibody production method according to the present invention is not limited thereto.

### (4) Method for preparing recombinant anti-APOA2 monoclonal antibody or fragment thereof by recombinant DNA manipulation

The antibody for use in the present invention can also be obtained by recombinant DNA manipulation using a cDNA sequence encoding the amino acid sequence of the antibody.

Nucleotide sequences encoding the amino acid sequences of the variable regions in the antibody derived from an anti-APOA2 monoclonal antibody-producing hybridoma, for example, the anti-APOA2 protein terminus monoclonal antibody-producing hybridoma obtained by the approach described in the paragraph "1-3-2(2)", are used. These nucleotide sequences of VH and VL are linked to nucleotide sequences encoding arbitrary human CH and human CL, respectively, and the resulting polynucleotides can be incorporated into appropriate expression vectors, which are then transferred to host cells, followed by expression as a complete immunoglobulin molecule. Alternatively, according to a CDR grafting antibody technique, polynucleotides encoding the respective amino acid sequences of the CDR sequences in the amino acid sequences of the variable regions in the antibody derived from the anti-APOA2 protein terminus monoclonal antibody-producing hybridoma obtained by the approach described in the paragraph "1-3-2(2)" may be linked to polynucleotides encoding the amino acid sequences of human FR sequences in a predetermined order, and the resultant can be incorporated into appropriate expression vectors, which are then transferred to host cells, followed by expression as a complete immunoglobulin molecule. In this respect, an approach is convenient in which the heavy chain and the light chain to be paired can be expressed in the same host cell and produced as a heavy chain/light chain dimer. Specifically, each cell is cotransfected with, for example, the light chain expression vector and the heavy chain expression vector, and the antibody according to the present invention can also be obtained from this transformed cell. Alternatively, polynucleotides encoding the amino acid sequences of the variable regions described above may be incorporated directly to appropriate expression vectors, which are then transferred to host cells, followed by expression as fragments of the immunoglobulin molecule. Alternatively, as mentioned above, polynucleotides respectively encoding VL and VH or the light chain and the heavy chain comprising the amino acid sequences may be linked via a nucleotide sequence encoding an appropriate linker, then incorporated to phages, and expressed as single-chain Fv or as a synthetic antibody fragment such as diabody. In addition, according to a recently developed phage display antibody technique (Brinkmann et al., 1995, J. Immunol. Methods, 182, 41-50; and International Publication Nos. WO97/13844 and WO90-02809), which involves expressing recombinant antibodies on phage surface by exploiting a gene engineering technique, single-chain Fv antibodies diversified by artificially shuffling genes encoding heavy and light chains can be expressed as phage-fusion proteins to obtain specific antibodies.

The methods for preparing the polynucleotide encoding the recombinant anti-APOA2 antibody or the fragment thereof, preparing vectors carrying the polynucleotide, and transferring the vectors to hosts can be carried out by use of a recombinant DNA technique known in the art. The recombinant anti-APOA2 protein antibody of interest or the fragment thereof can be obtained from the culture solution of the transformed cells or from the inside of the cells.

For example, plasmids, phagemids, cosmids, or virus vectors (e.g., SV40 virus based vector, EB virus based vector, and BPV based vector) can be used as immunoglobulin expression vectors, though the vectors are not limited thereto. For example, a BCMGS Neo vector, which is a BPV based vector, is a desirable vector for efficient expression of a foreign gene by the transformation of COS7 cells or the like therewith (Hajime Karasuyama, "Bovine papilloma virus vector", Masami Muramatsu and Hiroto Okayama, ed., Experimental Medicine, Suppl.: Handbook of Gene Engineering, 1991, Yodosha Co., Ltd., 297-299).

The vectors can comprise control elements (e.g., a promoter, an enhancer, a terminator, a polyadenylation site, and a splicing site) necessary for expressing the antibody or the fragment thereof, or an optional selective marker, in addition to the polynucleotide encoding the antibody or the fragment thereof.

The hosts described in the paragraph "1-2. Preparation of immunogen" as well as SP2/0 (mouse myeloma) cells (European Journal of Cancer Research Preview (1996) Vol. 5, 512-519; and Cancer Research (1990) Vol. 50, 1495-1502) are preferably used as the hosts for transformation.

In the present invention, the host cells comprising the vectors for the expression of the antibody or the fragment thereof can be cultured according to a routine method so that the antibody is produced into the culture supernatant or the host cells. Specifically, when the hosts are CHO cells, the host cells can be inoculated at 1 × 10⁵ cells/mL to a DMEM medium manufactured by Life Technologies Corp. (currently known as Thermo Fisher Scientific, Inc.) and cultured in a 5% CO₂ incubator of 37°C to obtain an antibody-comprising culture supernatant. When the host cells are, for example, *E. coli* cells, the host cells can be inoculated to a general medium for use in *E. coli* culture, such as an LB medium, and cultured for the induction of protein expression to produce the antibody into the culture supernatant or the host cells.

The expression product antibody or fragment thereof comprising constant regions can be purified and recovered from the culture supernatant or cell lysates using a protein A column, a protein G column, an anti-immunoglobulin antibody affinity column, or the like. By contrast, this purification method cannot be applied to the expression product consisting of variable regions without comprising constant regions. Therefore, other appropriate purification methods are applied thereto. The antibody or the fragment thereof can be expressed as a structure C-terminally fused with, for example, a tag sequence advantageous for purification, such as a histidine tag, and thereby purified by affinity chromatography using the corresponding ligand. Unless being the tag-fusion protein, such an antibody or a fragment thereof can be purified according to a routine protein purification method such as ammonium sulfate precipitation, ion-exchange chromatography, reverse-phase chromatography, gel filtration chromatography, or hydroxyapatite chromatography.

In order to confirm specificity for the particular APOA2 protein variant or the fragment thereof, the monoclonal antibody or the fragment thereof used in the present invention is preferably tested for cross-reactivity with the other variants before use, as mentioned above. The antigens for which the cross-reactivity of, for example, the anti-APOA2-ATQ protein terminus monoclonal antibody of the present invention or the fragment thereof should be confirmed are the APOA2-AT protein and the APOA2-A protein.

It is more preferred to confirm the cross-reactivity of the antibody or the fragment thereof used in the present invention with the proteins described above as well as other proteins having a partial structure in common with the APOA2 protein variants. ELISA with, for example, the APOA2-ATQ protein as an antigen may be used for confirming cross reaction. The antibody to be tested for reaction specificity, i.e., the anti-APOA2 protein terminus antibody or the fragment thereof, is reacted with the APOA2 protein variant in the presence of other antigen proteins for which the cross-reactivity should be confirmed. The cross-reactivity can be confirmed by observing the competition between the APOA2 protein variant and the antigen proteins. Such a method for confirming the cross-reactivity through the use of the principles of competitive inhibition eliminates the need of preparing reaction systems for all antigens and therefore permits rapid screening.

### 1-3-3. Structural confirmation of region in APOA2 protein recognized by obtained anti-APOA2 protein terminus monoclonal antibody

The type of the APOA2 protein variant specifically recognized by the obtained anti-APOA2 monoclonal antibody can be determined by preparing genes of various APOA2 protein variants using PCR reaction or the like on the basis of the gene of the APOA2 protein, and analyzing the binding activity of the monoclonal antibody against the various APOA2 protein variants obtained from the genes.

In the case of the anti-APOA2 protein terminus monoclonal antibody, such a method is specifically carried out as follows: first, the full-length APOA2 gene or varying lengths of fragments lacking 6 bases or 9 bases including the stop codon of the APOA2 gene from the stop codon toward the 5' end are prepared, and expression vectors having inserts of these fragments are prepared. Such a method for preparing the gene fragments having deletion mutation are described in "Zoku-Seikagaku Jikken Koza 1, Idenshi Kenkyuho II (Experiments in Biochemistry, second series, Methods in Gene research in English), p. 289-305, The Japanese Biochemical Society ed". Next, various APOA2 protein variants are prepared by the aforementioned method from host cells harboring the respective APOA2 protein variant expression vectors. Subsequently, the binding activity of the anti-APOA2 protein monoclonal antibody against the various APOA2 protein variants is evaluated by ELISA using these proteins as antigens. The monoclonal antibody can be determined as an anti-APOA2 protein terminus monoclonal antibody specifically binding to the particular APOA2 protein variant when the monoclonal antibody exhibits binding activity only against the particular variant and exhibits no or almost no binding activity against the other variants.

The APOA2 protein variant recognized by the obtained anti-APOA2 protein terminus monoclonal antibody can also be confirmed by a method as described below.

First, peptides having the sequences of the C-terminal regions of various APOA2 protein variants are each synthesized in a solid phase by a method known in the art. Subsequently, the binding activity of the anti-APOA2 protein terminus monoclonal antibody against the various peptides is evaluated by ELISA using these peptides as antigens. The anti-APOA2 protein monoclonal antibody can be determined as an anti-APOA2 protein terminus monoclonal antibody specifically binding to the particular APOA2 protein variant when the monoclonal antibody is found to have binding activity only against the peptide having the sequence of the particular C-terminal region.

### 1-4. Preparation of anti-APOA2 polyclonal antibody

The anti-APOA2 polyclonal antibody can be prepared by a method known in the art. Hereinafter, the method for obtaining an anti-APOA2 protein terminus antibody specifically binding to the particular APOA2 protein variant will be specifically given as an example.

### 1-4-1. Obtainment of antiserum

The anti-APOA2 protein terminus polyclonal antibody can be prepared in the same way as the method for preparing anti-APOA2 antibody-producing cells described in the paragraph 1-3-1(2). A C-terminal fragment having a length of at least 6 or more amino acids on the particular APOA2 protein variant sequence, for example, the peptide shown in SEQ ID NO: 28 or 29, can be used as an antigen. Antiserum comprising the polyclonal antibody recognizing the APOA2 protein can be recovered from the blood of the immunized animal 2 to 5 days, preferably 3 days, after the final immunization date.

1-4-2. Purification of anti-APOA2 terminus polyclonal antibody (1) Preparation of peptide-immobilized column

Affinity columns are prepared by respectively immobilizing the APOA2 protein C-terminal region peptide and a C-terminally amide group-added APOA2 protein C-terminal region peptide. The detailed method is described in "Experimental Protocol for Anti-Peptide Antibodies", the 2nd edition, Gakken Medical Shujunsha Co., Ltd. For example, formyl-Cellulofine or CNBr agarose carriers having functional groups capable of binding to amino groups of peptides, or carriers capable of binding to cysteine residues on peptide sequences via maleimide groups covalently bonded to the carriers can be used as carriers for use in the affinity columns. The length of the peptide to be immobilized is 6 or more amino acids, preferably 10 or more amino acids, more preferably 18 or more amino acids, further preferably 30 or more amino acids, including the C terminus of the APOA2 protein.

### (2) Antibody purification

The anti-APOA2 protein terminus polyclonal antibody can be purified from the antiserum using the peptide-immobilized affinity columns. For example, the antiserum is diluted with a suitable buffer solution. IgG antibodies comprised in the antiserum are adsorbed onto the APOA2 protein C-terminal region peptide-immobilized affinity column, and this adsorbed fraction is recovered. Subsequently, immunoglobulins exhibiting binding activity against a region other than the C-terminal region of the peptide are removed by adsorption using the C-terminally amidated APOA2 protein peptide-immobilized affinity column. Finally, the resulting non-adsorbed fraction is obtained as an anti-APOA2 protein terminus polyclonal antibody specifically recognizing the particular APOA2 protein variant.

### 2. Each step of method for assisting in determination of malignant pancreatic cystic tumor

A second embodiment of the method of the present invention relates to each step of a method for assisting in diagnosis of determining *in vitro* affection with malignant pancreatic cystic tumor. A feature of the present invention is measuring the amount of APOA2-AT protein or APOA2-ATQ protein present in a body fluid sample obtained from a test subject having pancreatic cystic tumor, and assisting in the determination of the pancreatic cystic tumor to be benign or malignant on the basis of the amount.

The method of the present invention comprises the steps of: measuring a marker for assisting in the determination of malignant pancreatic cystic tumor; and assisting in the determination of the pancreatic cystic tumor to be malignant or benign. Hereinafter, each step will be described in detail.

### 2-1. Step of measuring marker for assisting in determination of malignant pancreatic cystic tumor

The "step of measuring a marker for assisting in the determination of malignant pancreatic cystic tumor" is the step of measuring *in vitro* the amount of the marker for assisting in the determination of malignant pancreatic cystic tumor according to the present invention, i.e., APOA2-AT protein or APOA2-ATQ protein, or a fragment thereof present in a body fluid sample derived from the test subject.

In the present specification, the "test subject" refers to an individual determined to have pancreatic cystic tumor in need of determining the pancreatic cystic tumor to be benign or malignant. Affection with pancreatic cystic tumor can be determined by use of an imaging technique such as CT, MRI, MRCP, or EUS. A method for detecting pancreatic tumor using the concentrations of the APOA2-AT protein and the APOA2-ATQ protein in blood in combination is also known to be capable of detecting pancreatic cancer or benign pancreatic tumor including pancreatic cystic tumor (Patent Literature 2 and Non Patent Literature 4). Such an existing tumor marker may be used alone or in combination with an imaging technique. Examples of the method for detecting pancreatic tumor using the marker specifically include a method comprising the following steps:
a method for detecting pancreatic cancer or benign pancreatic tumor by measuring the amounts of APOA2 protein variants in a body fluid sample of a test subject, the method comprising steps (A) to (C):
(A) a first step of measuring the amount of the APOA2-ATQ protein in a sample using an anti-APOA2-ATQ terminus antibody that specifically binds to a carboxyl-terminal region of the APOA2-ATQ protein consisting of the amino acid sequence represented by SEQ ID NO: 2, and an anti-APOA2-ATQ non-terminus antibody binding to the amino acid sequence other than the carboxyl-terminal region;
(B) a second step of measuring the amount of the APOA2-AT protein in the sample using an anti-APOA2-AT terminus antibody that specifically binds to a carboxyl-terminal region of the APOA2-AT protein consisting of the amino acid sequence represented by SEQ ID NO: 1 and an anti-APOA2-AT non-terminus antibody binding to the amino acid sequence other than the carboxyl-terminal region; and
(C) a third step for determining the presence or absence of pancreatic cystic tumor by inputting, to a preset discriminant, the measurement value of the amount of APOA2-ATQ protein obtained in the first step and the measurement value of the amount of the APOA2-AT protein obtained in the second step, and comparing the resulting discriminant value with the discriminant value of a known normal subject.

The test subject having pancreatic cystic tumor may be determined by using an imaging technique and the detection method each alone or in combination.

In this context, examples of the individual include vertebrates. The individual is preferably a mammal, for example, a primate (a human, a monkey, a chimpanzee, an orangutan, a gorilla, etc.), a rodent (a mouse, a rat, a guinea pig, etc.), or an ungulate animal (cattle, a horse, sheep, a goat, etc.), more preferably a human. In the present specification, when the test subject is a human, this test subject is particularly referred to as a "human test subject" below.

In the present specification, the "body fluid sample" is a sample that is subjected to assisting in the determination of malignant pancreatic cystic tumor, and means a biological fluid. The body fluid sample is not particularly limited as long as the body fluid sample is a biological fluid possibly comprising the markers for assisting in the determination of the malignant pancreatic cystic tumor of the present invention. The body fluid sample includes, for example, blood, urine, lymphocyte culture supernatants, spinal fluid, digestive juice (including e.g., pancreatic juice, large intestine juice, esophageal gland secretions, and saliva), sweat, ascites, runny nose, tear, vaginal fluid, and semen. Blood or urine is preferred. In the present specification, the "blood" includes plasma and serum, and whole blood can also be preferably used. The whole blood is not limited by its type and can be, for example, venous blood, arterial blood, or umbilical cord blood. The body fluid sample may be a combination of two or more different body fluid samples obtained from the same individual. The method for detecting a malignant pancreatic cystic tumor according to the present invention permits detection even from low invasive blood or urine and is therefore very useful as a convenient detection method.

The "body fluid sample derived from a test subject" refers to a body fluid sample already collected from the test subject, and the act of collecting the body fluid sample is not included in the embodiment of the method of the present invention. The body fluid sample derived from a test subject may be subjected to the method of the present invention immediately after being collected from the test subject. Alternatively, the body fluid sample derived from a test subject may be refrigerated or frozen immediately after collection or after an appropriate treatment and brought to room temperature before being subjected to the method of the present invention. When the body fluid sample is blood, the appropriate treatment before refrigeration or freezing includes, for example, the anticoagulation treatment of collected whole blood by the addition of heparin or the like, followed by separation as plasma, and the coagulation treatment of whole blood by the addition of a coagulation accelerator or the like, followed by separation as serum. Such a treatment can be carried out on the basis of a technique known in the art.

In the present specification, the "amounts of the APOA2 protein variants" refer to the respective quantities of the two APOA2 protein variants present in the body fluid sample derived from a test subject. The quantities may be any of absolute and relative amounts. The absolute amounts correspond to the masses or volumes of the two APOA2 protein variants comprised in a predetermined amount of the body fluid sample. The relative amounts refer to, for example, relative measurement values of the two APOA2 protein variants derived from the test subject to the measurement values of standards used. Examples of the relative amounts include concentrations, fluorescence intensity, and absorbance.

The amounts of the APOA2 protein variants can be measured *in vitro* by use of a method known in the art. Examples thereof include a measurement method using substances capable of specifically binding to the two APOA2 protein variants, respectively. Alternative examples thereof include a method of directly measuring the amounts of the protein variants of interest by use of amino acid sequencing, mass spectrometry, electrophoresis, or the like. Hereinafter, a method using a specific binding substance widely used for protein quantification methods will be described as an example, though this method does not intend to limit the scope of the present invention.

In the present specification, the term "capable of specifically binding" means that a certain substance can substantially bind only to the particular APOA2 protein variant as a target of the present invention. In this case, the presence of nonspecific binding is acceptable without influencing the detection of the particular APOA2 protein variant.

Examples of the "substances capable of specifically binding" include APOA2-binding proteins. More specifically, the substances capable of specifically binding are, for example, "anti-APOA2 protein terminus antibodies" recognizing the difference in C-terminal region structure and binding to the APOA2 protein variants as their respective antigens, preferably "anti-human APOA2 protein terminus antibodies" each recognizing and binding to only one of the APOA2 protein variants when the human APOA2 protein variants comprising the amino acid sequence represented by SEQ ID NO: 1, 2 or 3 are used as the antigens, or binding fragments of these antibodies. Alternatively, their chemically modified derivatives may be used. In this context, the "chemically modified derivatives" also include, for example, the anti-APOA2 protein terminus antibodies or the antibody fragments thereof functionally modified as required for acquiring or maintaining the specific binding activity for the particular APOA2 protein variant, or the anti-APOA2 protein terminus antibodies or the binding fragments modified with labels necessary for detection. The functional modification is as mentioned above.

The antibodies for use in the assay of the APOA2 protein variants may be any of polyclonal and monoclonal antibodies. Monoclonal antibodies are preferred for achieving specific detection. For example, an anti-APOA2 protein terminus polyclonal antibody specifically binding to the APOA2 protein terminus can be prepared by the aforementioned method.

The two APOA2 protein variants can be assayed by an immunological method using the anti-APOA2 antibodies each binding only to the particular APOA2 protein variant. The immunological method may be any method using the anti-APOA2 antibodies and is preferably ELISA using the anti-APOA2 protein terminus antibodies as immobilized antibodies or labeled antibodies which are combined with another antibody binding to a region other than the APOA2 protein C terminus (anti-APOA2 protein non-terminus antibody). For example, the amount of the APOA2-AT protein can be measured by sandwich ELISA using the anti-APOA2-AT terminus antibody as a labeled antibody and using the anti-APOA2-AT non-terminus antibody as an immobilized antibody. The amount of the APOA2-ATQ protein can be measured by sandwich ELISA using the anti-APOA2-ATQ terminus antibody as an immobilized antibody and using the anti-APOA2-ATQ non-terminus antibody as a labeled antibody. The anti-APOA2 protein non-terminus antibody is commercially available from Abcam plc., Fitzgerald Industries International, or the like, and such a commercially available product may be used.

### 2-2. Step of determining affection

The "step of determining affection" is the step of assisting in the *in vitro* determination of the pancreatic cystic tumor to be benign or malignant on the basis of the amount of the protein measured in the step of measuring a marker for assisting in the determination of malignant pancreatic cystic tumor. The pancreatic cystic tumor can be determined to be benign or malignant by measuring the amount of the marker for assisting in the determination of malignant pancreatic cystic tumor, i.e., the amount of an APOA2 protein variant (amount of APOA2-AT protein or/and APOA2-ATQ protein) in a body fluid sample of the test subject having pancreatic cystic tumor, and assisting in the determination of malignant pancreatic cystic tumor. According to this determination step, any one of the amount of the APOA2-AT protein or the amount of the APOA2-ATQ protein may be used for the determination, and the pancreatic cystic tumor can be determined to be benign or malignant with high accuracy by combining the amounts of both the proteins. Hereinafter, each determination step will be described in detail.

The pancreatic cystic tumor can be determined to be benign or malignant on the basis of the amount of the APOA2-AT protein. Specifically, the amount of the APOA2-AT protein in a body fluid sample of the test subject is measured using an anti-APOA2-AT terminus antibody that specifically binds to a C-terminal region of the APOA2-AT protein consisting of the amino acid sequence represented by SEQ ID NO: 1, and an anti-APOA2-AT non-terminus antibody binding to an amino acid sequence other than the C-terminal region, and the determination of the test subject to have malignant pancreatic cystic tumor can be assisted in when the amount of APOA2-AT in the body fluid sample of the test subject having pancreatic cystic tumor is lower than that in a benign pancreatic cystic tumor patient. In this context, provided that the test subject can be determined to have malignant pancreatic cystic tumor, the test subject can be definitively diagnosed with malignant pancreatic cystic tumor and can be determined as a pancreatic cystic tumor patient in need of treatment such as tumor resection by surgical operation, by adding detailed imaging technique by CT, EUS, MRI, or the like, cystic fluid cytology by EUS-FNA, or scrutiny by pancreatic juice cytology or the like using ERCP.

The pancreatic cystic tumor can also be determined to be benign or malignant on the basis of the amount of the APOA2-ATQ protein. Specifically, the amount of the APOA2-ATQ protein is measured using an anti-APOA2-ATQ terminus antibody that specifically binds to a C-terminal region of the APOA2-ATQ protein consisting of the amino acid sequence represented by SEQ ID NO: 2, and an anti-APOA2-ATQ non-terminus antibody binding to an amino acid sequence other than the C-terminal region, and the determination of the test subject to have malignant pancreatic cystic tumor can be assisted in when the amount of APOA2-ATQ in the body fluid sample of the test subject having pancreatic cystic tumor is higher than that in a benign pancreatic cystic tumor patient. In this context as well, provided that the test subject can be determined to have malignant pancreatic cystic tumor, the test subject can be definitively diagnosed with malignant pancreatic cystic tumor and can be determined as a pancreatic cystic tumor patient in need of treatment such as tumor resection by surgical operation, by adding detailed imaging technique by CT, EUS, MRI, or the like, cystic fluid cytology by EUS-FNA, or scrutiny by ERCP.

The pancreatic cystic tumor can also be determined to be benign or malignant with higher accuracy by determining malignant pancreatic cystic tumor from only the amount of the APOA2-AT protein, further determining malignant pancreatic cystic tumor on the basis of the amount of the APOA2-ATQ protein, and combining the respective results. Specifically, the accuracy of the determination of the test subject to have malignant pancreatic cystic tumor can be enhanced by narrowing down the test subject having pancreatic cystic tumor to a test subject having a lower amount of the APOA2-AT protein in the body fluid sample than that in a benign pancreatic cystic patient and having a higher amount of the APOA2-ATQ protein than that in a benign pancreatic cystic patient. On the other hand, the accuracy of the determination of the test subject to have benign pancreatic cystic tumor can be enhanced when the amount of the APOA2-AT protein in the body fluid sample of the test subject having pancreatic cystic tumor is equivalent to or lower than that in a benign pancreatic cystic patient and the amount of the APOA2-ATQ protein is equivalent to or higher than that in a benign pancreatic cystic patient.

In the present invention, a cutoff value preset on the basis of the amount of the APOA2-AT protein in a known benign pancreatic cystic tumor patient group or malignant pancreatic cystic tumor patient group can be used in the comparison of the amount of the APOA2-AT protein in the body fluid sample of the test subject with that in a benign pancreatic cystic tumor patient. For example, the test subject can be determined to have malignant pancreatic cystic tumor when the measurement value of the APOA2-AT protein in the test subject is equal to or lower than the cutoff value set on the basis of the amount of the APOA2-AT protein in a known benign pancreatic cystic tumor patient group. Standard deviation can be used in the setting of the cutoff value, and the cutoff value is a value of "mean - 0.5 × standard deviation (SD)", more preferably a value of "mean - 1.0 × SD", further preferably a value of "mean - 2.0 × SD", particularly preferably a value of "mean - 3.0 × SD", of the measurement values of the benign pancreatic cystic tumor patient group. Alternatively, a percentile method may be used for the setting, and the cutoff value is a 30-percentile value, more preferably a 10-percentile value, further preferably a 5-percentile value, particularly preferably a 1-percentile value, of the measurement values of the benign pancreatic cystic tumor patient group.

The test subject can also be determined to have malignant pancreatic cystic tumor when the measurement value of the APOA2-AT protein in the test subject is equal to or lower than the cutoff value set on the basis of the amount of the APOA2-AT protein in a known malignant pancreatic cystic tumor patient group. Standard deviation can be used in the setting of the cutoff value, and the cutoff value is a value of "mean + 0.5 × SD", more preferably a value of "mean + 1.0 × SD", further preferably a value of "mean + 2.0 × SD", particularly preferably a value of "mean + 3.0 × SD", of the measurement values of the malignant pancreatic cystic tumor patient group. Alternatively, a percentile method may be used for the setting, and the cutoff value is a 70-percentile value, more preferably a 90-percentile value, further preferably a 95-percentile value, particularly preferably a 99-percentile value, of the measurement values of the malignant pancreatic cystic tumor patient group.

Likewise, in the present invention, a cutoff value preset on the basis of the amount of the APOA2-ATQ protein in a known benign pancreatic cystic tumor patient group or malignant pancreatic cystic tumor patient group can be used in the comparison of the amount of the APOA2-ATQ protein with that in a benign pancreatic cystic tumor patient. For example, the test subject can be determined to have malignant pancreatic cystic tumor when the measurement value of the APOA2-ATQ protein in the test subject is equal to or higher than the cutoff value set on the basis of the amount of the APOA2-ATQ protein in a known benign pancreatic cystic tumor patient group. Standard deviation can be used in the setting of the cutoff value, and the cutoff value is a value of "mean + 0.5 × standard deviation (SD)", more preferably a value of "mean + 1.0 × SD", further preferably a value of "mean + 2.0 × SD", particularly preferably a value of "mean + 3.0 × SD", of the measurement values of the benign pancreatic cystic tumor patient group. Alternatively, a percentile method may be used for the setting, and the cutoff value is a 70-percentile value, more preferably a 90-percentile value, further preferably a 95-percentile value, particularly preferably a 99-percentile value, of the measurement values of the benign pancreatic cystic tumor patient group.

The test subject can also be determined to have malignant pancreatic cystic tumor when the measurement value of the APOA2-ATQ protein in the test subject is equal to or higher than the cutoff value set on the basis of the amount of the APOA2-ATQ protein in a known malignant pancreatic cystic tumor patient group. Standard deviation can be used in the setting of the cutoff value, and the cutoff value is a value of "mean - 0.5 × standard deviation (SD)", more preferably a value of "mean - 1.0 × SD", further preferably a value of "mean - 2.0 × SD", particularly preferably a value of "mean - 3.0 × SD", of the measurement values of the malignant pancreatic cystic tumor patient group. Alternatively, a percentile method may be used for the setting, and the cutoff value is a 30-percentile value, more preferably a 10-percentile value, further preferably a 5-percentile value, particularly preferably a 1-percentile value, of the measurement values of the malignant pancreatic cystic tumor patient group.

In addition to the methods described above, statistical processing is used in determining the amount of the APOA2-AT protein of the test subject to be lower than that in a benign pancreatic cystic tumor patient. Only a test subject analyzed to have a statistically significantly "lower" value as compared with the value of the benign pancreatic cystic tumor patient may be determined to have a "lower value as compared with the value of the benign pancreatic cystic patient". Likewise, statistical processing is used in determining the amount of the APOA2-ATQ protein of the test subject to be higher than that in a benign pancreatic cystic tumor patient. Only a test subject analyzed to have a statistically significantly "higher" value as compared with the value of the benign pancreatic cystic tumor patient may be determined to have a "higher value as compared with the value of the benign pancreatic cystic tumor patient". Specific examples of the term "statistically significantly", for example, when the obtained value has a small critical value (significance level) include p < 0.05, p < 0.01, or p < 0.001. In this context, the term "p" or "p value" represents the probability of a statistical hypothesis being true by chance in the hypothesized distribution of statistics in a statistical test. Thus, smaller "p" or "p value" means that the hypothesis is closer to trueness. The phrase "statistically significantly different" means that there is a significant difference in the statistical processing of distinctive amounts of the marker for assisting in the determination of malignant pancreatic cystic tumor obtained from the test subject and the benign pancreatic cystic tumor patient. The test subject can be evaluated as having malignant pancreatic cystic tumor when being statistically significantly different as compared with the benign pancreatic cystic tumor patient. A test method known in the art capable of determining the presence or absence of significance can be appropriately used as the test method for the statistical processing without particular limitations. For example, a Student's test or multiple comparison test method can be used.

The concentration of the marker for assisting in the determination of malignant pancreatic cystic tumor in the body fluid sample of the benign pancreatic cystic tumor patient or the malignant pancreatic cystic tumor patient may be measured every time the concentration of the marker for assisting in the determination of malignant pancreatic cystic tumor in the body fluid sample of the test subject having pancreatic cystic tumor is measured. Alternatively, the concentration of the marker for assisting in the determination of malignant pancreatic cystic tumor measured in advance can also be used. Particularly, the concentration of the marker for assisting in the determination of malignant pancreatic cystic tumor is measured in advance under various physical conditions of benign pancreatic cystic tumor patients and malignant pancreatic cystic tumor patients. The values are input to a computer and databased. This approach is convenient because the concentration of the marker for assisting in the determination of malignant pancreatic cystic tumor in a benign pancreatic cystic tumor patient or a malignant pancreatic cystic tumor patient having the optimum physical conditions for comparison with the test subject can be used at once by merely inputting the physical conditions of the test subject into the computer.

In the present specification, the "AUC (area under the curve) value" means an area under a receiver operating characteristic curve (ROC curve) and serves as an index for measuring the accuracy of a prediction, determination, detection, or diagnosis method for classifying patients into a positive group and a negative group. In this curve, a value (false-positive rate) determined by subtracting the probability of producing positive results for positive patients (sensitivity) and the probability of producing negative results for negative patients (specificity) from 1 is plotted when the cutoff value of the marker is changed from a smaller value to a larger value. A higher value of AUC means higher accuracy of a diagnosis method.

In the present specification, the "sensitivity" means a value of (the number of true positives) / (the number of true positives + the number of false-negatives). Higher sensitivity can reduce oversight in malignant pathological conditions in pancreatic cystic tumor patients and leads to comprehensive narrowing of malignant pancreatic cystic tumor patients.

In the present specification, the "specificity" means a value of (the number of true negatives) / (the number of true negatives + the number of false-positives). Higher specificity prevents needless additional tests or treatments ascribable to the misjudgment of benign pancreatic cystic tumor patients as malignant pancreatic cystic tumor patients and leads to the alleviation of burdens on patients or reduction in medical cost.

### 3. Kit for assisting in determination of malignant pancreatic cystic tumor

The "kit for assisting in the determination of malignant pancreatic cystic tumor" used in the present invention refers to a kit that is directly or indirectly used for assisting in the determination of pancreatic cystic tumor to be benign or malignant. The kit of the present invention is a kit for use in the method of the present invention, and detailed conditions such as measurement principles are the same as those described in the above sections 1. and 2. unless otherwise specified below or unless contradicted. The kit for assisting in the determination of malignant pancreatic cystic tumor according to the present invention is specifically a kit for measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, comprised in a body fluid sample obtained from a test subject.

The kit of the present invention is not particularly limited by its form as long as the kit can detect the APOA2-AT protein or/and the APOA2-ATQ protein in a body fluid sample obtained from a test subject. The kit may be, for example, a kit for immunoassay comprising antibodies against the APOA2 protein variants as constituents. Examples of the immunoassay include enzyme immunoassay (EIA) (e.g., direct competitive ELISA, indirect competitive ELISA, and sandwich ELISA), radioimmunoassay (RIA), latex agglutination method, fluorescence immunoassay (FIA), chemiluminescence enzyme immunoassay (CLEIA), chemiluminescence immunoassay (CLIA), turbidimetric immunoassay (TIA), and immunochromatography. The kit of the present invention is particularly preferably a kit for sandwich ELISA.

The antibodies against the APOA2 protein variants are specifically antibodies including an anti-APOA2-AT terminus antibody or/and an anti-APOA2-ATQ terminus antibody or/and their binding fragments. In the form of a kit for ELISA, each of the anti-APOA2-AT terminus antibody and the anti-APOA2-ATQ terminus antibody may be in the state of an antibody solution comprising the antibody dissolved alone in a buffer solution or may be dissolved in a buffer solution and prepared into a frozen or freeze-dried state. The antibodies may each be in a state immobilized on the surface of a solid phase such as a microwell plate or beads. Alternatively, the antibodies may each be labeled. In this case, in a kit comprising both the anti-APOA2-AT terminus and anti-APOA2-ATQ terminus antibodies, it is preferred that both the antibodies should be labeled in different manners. The buffer solution or the solid phase surface comprising each of the antibodies preferably further comprises a stabilizer (e.g., albumin and sugars) or a preservative (e.g., sodium azide).

The anti-APOA2-ATQ terminus antibody is a monoclonal antibody that specifically binds to a carboxyl-terminal region of the APOA2-ATQ protein consisting of the amino acid sequence represented by SEQ ID NO: 2, and preferably has heavy chain CDR1, CDR2, and CDR3 consisting of the amino acid sequences represented by SEQ ID NOs: 4, 5, and 6 or 10, 11, and 12, respectively, and light chain CDR1, CDR2, and CDR3 consisting of the amino acid sequences represented by SEQ ID NOs: 7, 8, and 9 or 13, 14, and 15, respectively.

The anti-APOA2-AT terminus antibody is a monoclonal antibody that specifically binds to a carboxyl-terminal region of the APOA2-AT protein consisting of the amino acid sequence represented by SEQ ID NO: 1, and preferably has heavy chain CDR1, CDR2, and CDR3 consisting of the amino acid sequences represented by SEQ ID NOs: 33, 34, and 35, SEQ ID NOs: 39, 40, and 41, or SEQ ID NOs: 45, 46, and 47, respectively, and light chain CDR1, CDR2, and CDR3 consisting of the amino acid sequences represented by SEQ ID NOs: 36, 37, and 38, SEQ ID NOs: 42, 43, and 44, or SEQ ID NOs: 48, 49, and 50, respectively.

The kit for ELISA may further comprise, as other constituents, an antibody, a reagent, an instrument, and the like necessary for detecting APOA2-AT or/and APOA2-ATQ in a body fluid sample obtained from a test subject. Specific examples thereof include a test solution comprising each anti-APOA2 non-terminus antibody, a washing solution, and a test solution comprising an enzyme substrate in the case of using an enzyme label. For example, in the case of immobilizing the anti-APOA2-AT terminus antibody or/and the anti-APOA2-ATQ terminus antibody on a solid phase, each anti-APOA2 non-terminus antibody is preferably labeled, and the labeled anti-APOA2 non-terminus antibody is preferably in the state of an antibody solution comprising the antibody dissolved in a buffer solution.

The anti-APOA2 non-terminus antibody is a monoclonal antibody that specifically binds to an amino acid sequence other than the carboxyl-terminal region of the APOA2-AT protein consisting of the amino acid sequence represented by SEQ ID NO: 1 or the APOA2-ATQ protein consisting of the amino acid sequence represented by SEQ ID NO: 2, and preferably has heavy chain CDR1, CDR2, and CDR3 consisting of the amino acid sequences represented by SEQ ID NOs: 16, 17, and 18 or 22, 23, and 24, respectively, and light chain CDR1, CDR2, and CDR3 consisting of the amino acid sequences represented by SEQ ID NOs: 19, 20, and 21 or 25, 26, and 27, respectively.

The kit of the present invention is not limited to the kit for ELISA and may be in other forms as described above. In ELISA and other forms, all the constituents necessary for the kit are well known to those skilled in the art.

### Examples

Hereinafter, the present invention will be described further specifically with reference to Examples. However, the present invention is not intended to be limited by these Examples.

### (Example 1) Preparation of monoclonal antibody specifically recognizing C-terminal region of APOA2-AT protein

### (a) Preparation of cell producing antibody recognizing C-terminal region of APOA2-AT protein

A peptide consisting of the amino acid sequence represented by SEQ ID NO: 28, which is the sequence of a C-terminal region of the APOA2-AT protein, is poorly soluble in water and low antigenic. Therefore, hydrophilicity was imparted thereto by the addition of 3 arginine residues to the N-terminal side, and a cysteine residue was further added to the N terminus to synthesize an AT peptide. For specificity evaluation, three arginine residues were added to the N-terminal side of a peptide consisting of the amino acid sequence represented by SEQ ID NO: 29, which is the sequence of a C-terminal region of the APOA2-ATQ protein, to synthesize an ATQ peptide.

Subsequently, KLH protein was bound to the cysteine residue of the AT peptide. The resulting peptide was mixed as an immunogen with Sigma Adjuvant System (manufactured by Sigma-Aldrich Corp.) such that the amount of the immunogen was 50 to 100 µg per mouse. The immunogen was intraperitoneally administered to mice (BALB/c) five times at 2-week intervals.

During the immunization period, blood was collected from the tail vein over time, and an antibody titer in the mouse blood was confirmed using the obtained plasma. A protein consisting of the peptide bound with bovine serum albumin was adjusted to 3 µg/mL with a PBS solution, then added at 50 µL/well to Immunoplate Maxisorp (manufactured by Thermo-Fisher Scientific Inc.), and immobilized overnight. The solution was discarded, and Blocking One (manufactured by Nacalai Tesque, Inc.) was added thereto at 250 µL/well. The plate was blocked at room temperature for 1 hour or longer. The solution in each well was discarded. Then, the well was washed by the addition of PBS-T (0.05% Tween(R) 20 and PBS). The mouse serum diluted with PBS-T was added thereto at 50 µL/well and reacted at room temperature for 2 hours. The solution in each well was discarded. Then, the well was washed with PBS-T. Polyclonal Rabbit Anti-Mouse Immunoglobulins/HRP (manufactured by GE Healthcare Life Sciences (currently known as Cytiva)) diluted 5000-fold with PBS-T was added thereto at 50 µL/well and reacted at room temperature for 1 hour. After washing with PBS-T, a TMB solution (manufactured by Kirkegaard & Perry Laboratories, Inc. (KPL)) was added thereto at 50 µL/well for enzymatic reaction. Then, the reaction was terminated by the addition of a 2 N sulfuric acid solution at 50 µL/well. The absorbance was measured at 450 nm. As a result, a rise in antibody titer was able to be confirmed. Therefore, lymph nodes were harvested and subcutaneously transplanted to the kidney of immunodeficient mice (C.B.17/SCID). The mice were boosted three times, and a rise in antibody titer in blood was confirmed. Then, the spleen was harvested, and antibody-producing cells were separated therefrom.

### (b) Recovery of antibody-producing cell from mouse and cell fusion

The antibody-producing cells were washed twice in an RPMI1640 medium, then mixed with SP2/0 (mouse myeloma) cells, and subjected to fusion reaction using PEG1500 (manufactured by Roche Diagnostics K.K.). Subsequently, the fusion cells were cultured for approximately 1 week in a HAT medium and cloned.

### (c) Selection of hybridoma producing antibody recognizing C-terminal region of APOA2-AT protein

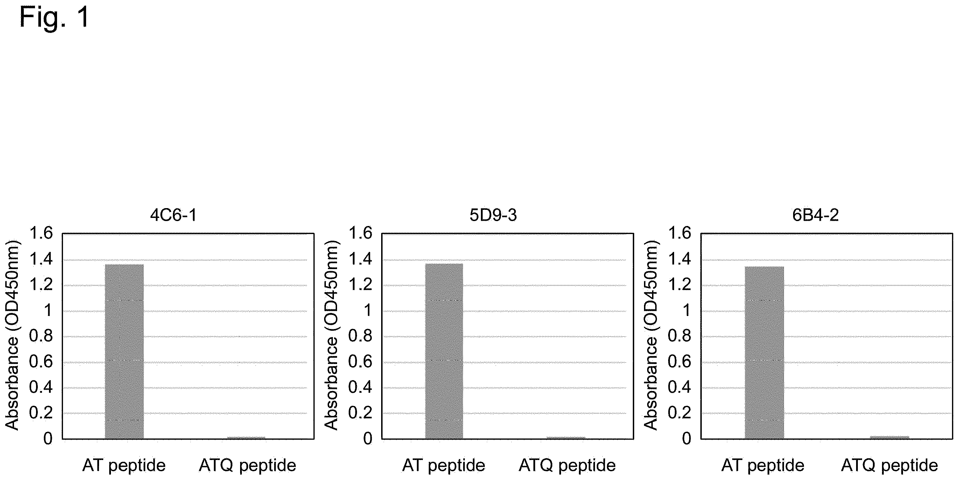

Next, using a culture supernatant of the hybridomas selected in a HAT medium, antibodies specifically recognizing the C-terminal region of the APOA2-AT protein were selected by using, as an index, difference in binding activity against the AT peptide and the ATQ peptide. Bovine serum albumin bound with the AT peptide or bovine serum albumin bound with the ATQ peptide was adjusted to 3 µg/mL with a PBS solution, then added at 50 µL/well to Immunoplate Maxisorp, and immobilized overnight. As a result of screening by ELISA in the same way as in the step (a), three hybridomas, clones 4C6-1, 5D9-3, and 6B4-2, specifically reacting only with the AT peptide, were obtained. Figure 1 shows the binding activity of the clones 4C6-1, 5D9-3, and 6B4-2 against the APOA2-AT peptide and the APOA2-ATQ peptide.

As a result of sequence analysis on genes encoding these monoclonal antibodies, the CDR sequences of 4C6-1 were shown to be the amino acid sequences represented by SEQ ID NOs: 33 to 38; the CDR sequences of 5D9-3 were shown to be the amino acid sequences represented by SEQ ID NOs: 39 to 44; and the CDR sequences of 6B4-2 were shown to be the amino acid sequences represented by SEQ ID NOs: 45 to 50.

### (Example 2) Detection of APOA2-AT protein by sandwich ELISA

The monoclonal antibody 4C6-1, 5D9-3, or 6B4-2 obtained in Example 1 or an anti-APOA2-AT terminus polyclonal antibody and anti-APOA2 non-terminus monoclonal antibody MAB1 were used to detect the APOA2-AT protein by sandwich ELISA.

In this study, each anti-APOA2-AT terminus antibody was labeled with POD using PEROXIDASE LABELING KIT-SH (manufactured by Dojindo Laboratories Co., Ltd.), and the details abided by the attached protocol. The anti-APOA2 non-terminus antibody was adjusted to 4 µg/mL with a PBS solution, then added at 100 µL/well to Immunoplate Maxisorp, and immobilized overnight. On the next day, the solution was discarded, and the well was washed by the addition of 400 µL of PBS-T. A blocking buffer solution (1% BSA, 0.05% Tween 20, and PBS) was added thereto at 400 µL/well. The plate was left standing overnight at 4°C. Then, the solution was discarded to prepare an antibody-immobilized plate. Next, the APOA2-AT protein serially diluted with a diluent was added thereto at 100 µL/well and reacted at room temperature for 1 hour. Then, the solution in the well was discarded. Then, the well was washed with PBS-T. The POD-labeled anti-APOA2-AT terminus antibody diluted with a diluent was added thereto at 100 µL/well and reacted at room temperature for 1 hour. After washing with PBS-T, a TMB solution was added thereto at 100 µL/well for enzymatic reaction. Finally, the reaction was terminated by the addition of a 2 N sulfuric acid solution at 100 µL/well. The absorbance was measured at 450 nm.

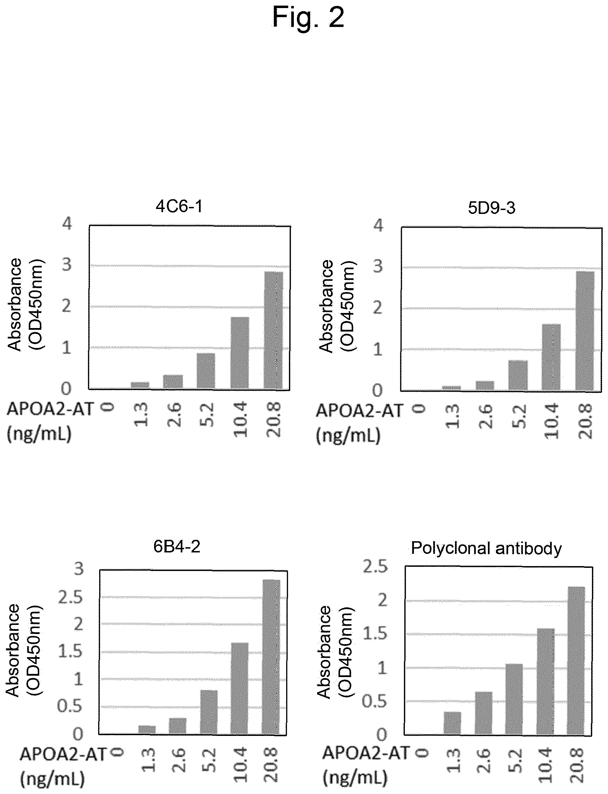

Figure 2 shows results of detecting each concentration of the APOA2-AT protein by sandwich ELISA using anti-APOA2 non-terminus monoclonal antibody MAB 1 and each anti-APOA2-AT terminus monoclonal antibody or an anti-APOA2-AT terminus polyclonal antibody. As shown in Figure 2, increase in absorbance was confirmed in a manner dependent on the concentration of the APOA2-AT protein. This demonstrated that change in the concentration of the APOA2AT protein can be detected by use of ELISA.

In the following Examples and Comparative Examples, the performance of a method for determining a malignant pancreatic cystic tumor group and a benign pancreatic cystic tumor group using the APOA2-AT protein, the APOA2-ATQ protein, and CA19-9 was tested using plasma obtained from patients with intraductal papillary mucinous neoplasm (IPMN), a pancreatic cystic tumor.

### (Example 3) Determination of malignant pancreatic cystic tumor group using two APOA2 variants

### (i) Plasma sample of IPMN patient

The concentrations of the APOA2-AT protein and the APOA2-ATQ protein were measured by ELISA targeting plasma collected with informed consent from 15 benign IPMN patients and 7 malignant IPMN patients.

### (ii) Measurement of amount of APOA2-AT protein

The amount of the APOA2-AT protein in plasma was measured by sandwich ELISA using an anti-APOA2-AT terminus polyclonal antibody and the anti-APOA2 non-terminus monoclonal antibody MAB1 labeled with POD targeting the plasma described above. The anti-APOA2 non-terminus monoclonal antibody was labeled with POD using PEROXIDASE LABELING KIT-SH, and the details abided by the attached protocol. The anti-APOA2-AT terminus polyclonal antibody was adjusted to 2 µg/mL with a PBS solution, then added at 100 µL/well to Immunoplate Maxisorp (manufactured by Nunc/Thermo Fisher Scientific Inc.), and immobilized overnight. On the next day, the solution was discarded, and the well was washed by the addition of 400 µL of PBS-T (0.05% Tween 20 and PBS). A blocking buffer (1% BSA, 0.05% Tween 20, and PBS) solution was added thereto at 400 µL/well. The plate was left standing at room temperature for 1 hour. Then, the solution was discarded to prepare an antibody-immobilized plate. Next, the plasma diluted with a diluent was added thereto at 100 µL/well and reacted at room temperature for 1 hour. In this respect, the dilution ratio of the plasma was set to 10000-fold. The antigen solution in the well was discarded. Then, the well was washed with PBS-T. The POD-labeled anti-APOA2 non-terminus polyclonal antibody diluted to 0.2 µg/mL with a diluent was added thereto at 100 µL/well and reacted at room temperature for 1 hour. After washing with PBS-T (manufactured by Pierce Biotechnology, Inc. (currently known as Thermo-Fisher Scientific Inc.)), a TMB solution was added thereto at 100 µL/well for enzymatic reaction. Finally, the reaction was terminated by the addition of a 0.5 N sulfuric acid solution at 100 µL/well. The absorbance was measured at 450 nm. The protein concentration in blood was calculated as to the obtained measurement value by using, as a preparation, an antigen solution of recombinant human-derived APOA2-AT protein.

### (iii) Measurement of amount of APOA2-ATQ protein

The amount of the APOA2-ATQ protein in plasma was measured by sandwich ELISA using anti-APOA2-ATQ terminus monoclonal antibody 7F2 labeled with POD and the anti-APOA2 non-terminus polyclonal antibody in the same way as in the measurement of the amount of the APOA2-AT protein targeting the same plasma as above. The labeling of the antibody 7F2 with POD and the sandwich ELISA were also performed in the same way as above. The protein concentration in blood was calculated as to the obtained measurement value by using, as a preparation, an antigen solution of recombinant human-derived APOA2-ATQ protein.

### (iv) Determination of malignant pancreatic cystic tumor group

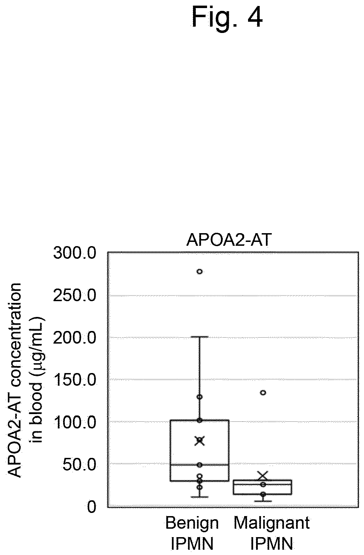

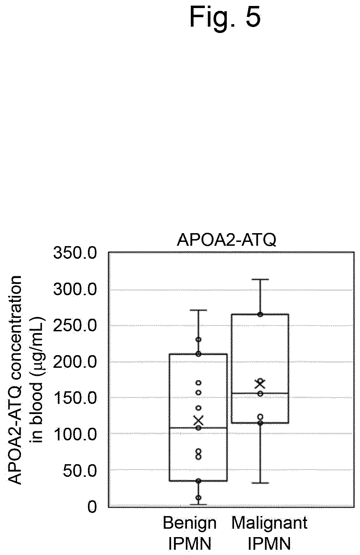

Figures 4 and 5 each show results of plotting the measurement values of the APOA2-AT protein and the APOA2-ATQ protein in each sample in a box-and-whisker plot. A mean of the APOA2-AT protein concentrations was found lower in the malignant group compared with the benign group, and a mean of the APOA2-ATQ protein concentrations was found higher in the malignant group compared with the benign group.

Table 1 shows concentrations in each plasma sample of 7 cases of malignant IPMN patients and two types of cutoff values calculated from concentrations in the plasma of 15 cases of benign IPMN patients or 7 cases of malignant IPMN patients, as to the APOA2-AT protein and the APOA2-ATQ protein. Cutoff value A of the APOA2-AT protein calculated from the concentrations in the plasma of 15 cases of benign IPMN patients was set to a value (39.7 µg/ml) obtained by subtracting 0.5 x SD from a mean of the concentrations in 15 plasma samples of the benign IPMN patients. Cutoff value B of the APOA2-AT protein calculated from the concentrations in the plasma of 7 cases of malignant IPMN patients was set to a value (57.9 µg/ml) obtained by adding 0.5 x SD to a mean of the concentrations in 7 plasma samples of the malignant IPMN patients. Cutoff value A of the APOA2-ATQ protein calculated from the concentrations in the plasma of 15 cases of benign IPMN patients was set to a value (162.2 µg/ml) obtained by adding 0.5 x SD to a mean from the benign IPMN patients. Cutoff value B of the APOA2-ATQ protein calculated from the concentrations in the plasma of 7 cases of malignant IPMN patients was set to a value (120.7 µg/ml) obtained by subtracting 0.5 x SD from a mean of the concentrations in 7 plasma samples of the malignant IPMN patients.

In the case of using cutoff value A calculated from the concentrations in the plasma of 15 cases of benign IPMN patients, 6 cases (malignant A, malignant C, malignant D, malignant E, malignant F, and malignant G) among 7 malignant IPMN cases fell below the cutoff value in the determination with the APOA2-AT protein and were correctly determined to be malignant, and 3 cases (malignant A, malignant C, and malignant D) exceeded the cutoff value in the determination with the APOA2-ATQ protein and were correctly determined to be malignant. In the case of using cutoff value B calculated from the concentrations in the plasma of 7 cases of malignant IPMN patients, 6 cases (malignant A, malignant C, malignant D, malignant E, malignant F, and malignant G) among 7 malignant IPMN cases fell below the cutoff value in the determination with the APOA2-AT protein and were correctly determined to be malignant, and 5 cases (malignant A, malignant C, malignant D, malignant E, and malignant F) exceeded the cutoff value in the determination with the APOA2-ATQ protein and were correctly determined to be malignant.

### (Comparative Example 1) Discrimination of malignant pancreatic cystic tumor group from benign pancreatic cystic tumor group using CA19-9

### (i) Plasma sample of IPMN patient

The concentration of CA19-9 in plasma was measured by ELISA targeting the 15 cases of benign IPMN patients and the 7 cases of malignant IPMN patients used in Example 3.

### (ii) Determination of malignant pancreatic cystic tumor group using CA19-9

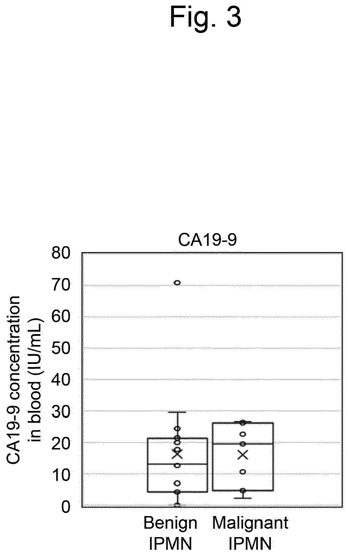

Figure 3 shows results of plotting the measurement value of each sample in a box-and-whisker plot. A mean of the CA19-9 concentrations was found equivalent between the benign group and the malignant group. Table 1 shows concentrations in each plasma sample of 7 cases of malignant IPMN patients and two types of cutoff values calculated from concentrations in the plasma of 15 cases of benign IPMN patients or 7 cases of malignant IPMN patients, as to CA19-9. Cutoff value A was set to a value (25.3 U/ml) obtained by adding 0.5 x SD to a mean from the benign IPMN patients, and cutoff value B was set to a value (11.1 U/ml) obtained by subtracting 0.5 x SD from a mean from the malignant IPMN patients.

In the case of using cutoff value A calculated from the concentrations in the plasma of 15 cases of benign IPMN patients, only 2 cases (malignant C and malignant G) among 7 malignant IPMN cases exceeded the cutoff value and were determined to be malignant. In the case of using cutoff value B calculated from the concentrations in the plasma of 7 cases of malignant IPMN patients, 4 cases (malignant B, malignant C, malignant F, and malignant G) among 7 malignant IPMN cases exceeded the cutoff value and were determined to be malignant.

The results of Example 3 and Comparative Example 1 described above demonstrated that both the APOA2-AT protein and the APOA2-ATQ protein enable malignant pancreatic cystic tumor to be detected with higher sensitivity than that of CA19-9.

**[Table 1]**

| Sample | APOA2-AT concentration (µg/ml) | APOA2-ATQ concentration (µl/ml) | CA19-9 (U/ml) |
|---|---|---|---|
| Malignant A | 5.3 | 314.4 | 10.7 |
| Malignant B | 134.5 | 31.7 | 22.7 |
| Malignant C | 25.5 | 265.5 | 26.7 |
| Malignant D | 15.4 | 173.3 | 4.7 |
| Malignant E | 25.4 | 123.2 | 2.3 |
| Malignant F | 13.8 | 155.4 | 19.6 |
| Malignant G | 30.0 | 115.1 | 26.4 |
| Mean from 7 malignant cases | 35.7 | 168.4 | 16.2 |
| Mean from 15 benign cases | 77.3 | 118.3 | 16.4 |
| Cutoff value A | 39.7 | 162.2 | 25.3 |
| Cutoff value B | 57.9 | 120.7 | 11.1 |

### (Comparative Example 2) Discrimination of malignant pancreatic cystic tumor group from benign pancreatic cystic tumor group using CA19-9

Following Comparative Example 1, discrimination performance was evaluated in 15 cases of benign IPMN patients and 7 cases of malignant IPMN patients using CA19-9. As a result of ROC analysis, the AUC value was 0.58, and the discrimination performance was thus confirmed to be low.

### (Example 4) Discrimination of malignant pancreatic cystic tumor group from benign pancreatic cystic tumor group using APOA2 protein variant in blood

Following Example 3, discrimination performance was evaluated in 15 cases of benign IPMN patients and 7 cases of malignant IPMN patients using the APOA2-AT protein and the APOA2-ATQ protein as respective markers in blood. As a result of ROC analysis, the discrimination performance of the APOA2-AT protein was AUC value = 0.762, and the discrimination performance of the APOA2-ATQ protein was AUC value = 0.648. Thus, these proteins were confirmed to achieve discrimination with high accuracy as compared with the discrimination method using CA19-9.

The results of Examples and Comparative Examples described above revealed that the method for assisting in the determination of malignant pancreatic cystic tumor using the APOA2-AT protein and the APOA2-ATQ protein according to the present invention is useful in the discrimination of malignant pancreatic cystic tumor from benign pancreatic cystic tumor by a noninvasive method regarded as being difficult so far.

### Industrial Applicability

According to the present invention, the determination of pancreatic cystic tumor to be benign or malignant can be assisted in by a simple and noninvasive method, and malignant pancreatic cystic tumor to be scrutinized or treated can be found. The present invention is an invention having wide applicability in the medical field, the pharmaceutical field, and the like.

All publications, patents, and patent applications cited herein are incorporated herein by reference in their entirety.

## Claims

1. A method for assisting in the determination of malignant pancreatic cystic tumor in a test subject having pancreatic cystic tumor, the method comprising the steps of measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, present in a body fluid sample obtained from the test subject, and assisting in the determination of the pancreatic cystic tumor of the test subject to be benign or malignant on the basis of the amount.

2. The method according to claim 1, wherein the pancreatic cystic tumor is intraductal papillary mucinous neoplasm (IPMN) or mucinous cystic neoplasm (MCN).

3. The method according to claim 1 or 2, wherein the determination of the pancreatic cystic tumor to be malignant is assisted in when the amount of the APOA2-AT protein present in the body fluid sample obtained from the test subject is lower than the amount of the APOA2-AT protein present in a body fluid sample obtained from a test subject with known benign pancreatic cystic tumor.

4. The method according to claim 3, wherein the determination of the pancreatic cystic tumor to be malignant is assisted in when the amount of the APOA2-AT protein present in the body fluid sample obtained from the test subject is lower than a cutoff value set on the basis of the amount of the APOA2-AT protein present in a body fluid sample obtained from a test subject with known benign pancreatic cystic tumor or a cutoff value set on the basis of the amount of the APOA2-AT protein present in a sample obtained from a test subject with known malignant pancreatic cystic tumor.

5. The method according to any one of claims 1 to 4, wherein the determination of the pancreatic cystic tumor to be malignant is assisted in when the amount of the APOA2-ATQ protein present in the body fluid sample obtained from the test subject is higher than the amount of the APOA2-ATQ protein present in a body fluid sample obtained from a test subject with known benign pancreatic cystic tumor.

6. The method according to claim 5, wherein the determination of the pancreatic cystic tumor to be malignant is assisted in when the amount of the APOA2-ATQ protein present in the body fluid sample obtained from the test subject is higher than a cutoff value set on the basis of the amount of the APOA2-ATQ protein present in a body fluid sample obtained from a test subject with known benign pancreatic cystic tumor or a cutoff value set on the basis of the amount of the APOA2-ATQ protein present in a sample obtained from a test subject with known malignant pancreatic cystic tumor.

7. The method according to any one of claims 1 to 6, wherein the body fluid sample is blood, plasma, or serum.

8. The method according to any one of claims 1 to 7, wherein the test subject is determined in advance to have pancreatic cystic tumor by an imaging technique and/or the following steps (A) to (C):
(A) a first step of measuring the amount of the APOA2-ATQ protein in a sample using an anti-APOA2-ATQ terminus antibody that specifically binds to a carboxyl-terminal region of the APOA2-ATQ protein consisting of the amino acid sequence represented by SEQ ID NO: 2, and an anti-APOA2-ATQ non-terminus antibody binding to the amino acid sequence other than the carboxyl-terminal region;
(B) a second step of measuring the amount of the APOA2-AT protein in the sample using an anti-APOA2-AT terminus antibody that specifically binds to a carboxyl-terminal region of the APOA2-AT protein consisting of the amino acid sequence represented by SEQ ID NO: 1 and an anti-APOA2-AT non-terminus antibody binding to the amino acid sequence other than the carboxyl-terminal region; and
(C) a third step for determining the presence or absence of pancreatic cystic tumor by inputting, to a preset discriminant, the measurement value of the amount of APOA2-ATQ protein obtained in the first step and the measurement value of the amount of the APOA2-AT protein obtained in the second step, and comparing the resulting discriminant value with the discriminant value of a known normal subject.

9. A kit for assisting in the determination of malignant pancreatic cystic tumor, the kit being directed to measuring *in vitro* the amount of APOA2-AT protein which is a polypeptide comprising the amino acid sequence represented by SEQ ID NO: 30 at the carboxyl terminus or/and APOA2-ATQ protein which is a polypeptide comprising the amino acid sequence represented by SEQ ID NO: 31 at the carboxyl terminus, comprised in a body fluid sample obtained from a test subject.

10. The kit according to claim 9, comprising an anti-APOA2-AT terminus antibody or a binding fragment thereof, or/and an anti-APOA2-ATQ terminus antibody or a binding fragment thereof.

11. The kit according to claim 10, wherein the anti-APOA2-ATQ terminus antibody or the binding fragment thereof is an anti-APOA2-ATQ terminus monoclonal antibody that specifically binds to a carboxyl-terminal region of the APOA2-ATQ protein consisting of the amino acid sequence represented by SEQ ID NO: 2, and has heavy chain CDR1, CDR2, and CDR3 consisting of the amino acid sequences represented by SEQ ID NOs: 4, 5, and 6 or 10, 11, and 12, respectively, and light chain CDR1, CDR2, and CDR3 consisting of the amino acid sequences represented by SEQ ID NOs: 7, 8, and 9 or 13, 14, and 15, respectively, or a binding fragment thereof.

12. The kit according to claim 10 or 11, wherein the anti-APOA2-AT terminus antibody or the binding fragment thereof comprises an anti-APOA2-AT terminus monoclonal antibody that specifically binds to a carboxyl-terminal region of the APOA2-AT protein consisting of the amino acid sequence represented by SEQ ID NO: 1, and has heavy chain CDR1, CDR2, and CDR3 consisting of the amino acid sequences represented by SEQ ID NOs: 33, 34, and 35, SEQ ID NOs: 39, 40, and 41, or SEQ ID NOs: 45, 46, and 47, respectively, and light chain CDR1, CDR2, and CDR3 consisting of the amino acid sequences represented by SEQ ID NOs: 36, 37, and 38, SEQ ID NOs: 42, 43, and 44, or SEQ ID NOs: 48, 49, and 50, respectively, or a binding fragment thereof.

13. The kit according to any one of claims 10 to 12, further comprising an anti-APOA2 non-terminus monoclonal antibody that specifically binds to an amino acid sequence other than the carboxyl-terminal region of the APOA2-AT protein consisting of the amino acid sequence represented by SEQ ID NO: 1 or the APOA2-ATQ protein consisting of the amino acid sequence represented by SEQ ID NO: 2, and has heavy chain CDR1, CDR2, and CDR3 consisting of the amino acid sequences represented by SEQ ID NOs: 16, 17, and 18 or 22, 23, and 24, respectively, and light chain CDR1, CDR2, and CDR3 consisting of the amino acid sequences represented by SEQ ID NOs: 19, 20, and 21 or 25, 26, and 27, respectively, or a binding fragment thereof.

14. An anti-APOA2-AT terminus monoclonal antibody that specifically binds to a carboxyl-terminal region of the APOA2-AT protein consisting of the amino acid sequence represented by SEQ ID NO: 1, and has heavy chain CDR1, CDR2, and CDR3 consisting of the amino acid sequences represented by SEQ ID NOs: 33, 34, and 35, SEQ ID NOs: 39, 40, and 41, or SEQ ID NOs: 45, 46, and 47, respectively, and light chain CDR1, CDR2, and CDR3 consisting of the amino acid sequences represented by SEQ ID NOs: 36, 37, and 38, SEQ ID NOs: 42, 43, and 44, or SEQ ID NOs: 48, 49, and 50, respectively, or a binding fragment thereof.
